# EUROPÄISCHE PATENTANMELDUNG

(11) **EP 4 286 368 A1**
(43) Veröffentlichungstag der Anmeldung: **06.12.2023**
(21) Anmeldenummer: 22176467.3
(22) Anmeldetag: 31.05.2022
(51) Int. Cl.: C07C 253/30, C07C 255/56, C07C 303/02, C07C 309/17, C07D 471/04

(54) **VERFAHREN ZUR HERSTELLUNG VON 4-FORMYL-3-METHOXYBENZONITRIL**

(71) Anmelder: Bayer Aktiengesellschaft, 51373 Leverkusen (DE)
(72) Erfinder: Platzek, Johannes, 12621 Berlin (DE)
(74) Vertreter: BIP Patents

(57) **Zusammenfassung**

Die vorliegende Erfindung betrifft ein Verfahren zur Herstellung von 4-Formyl-3-methoxybenzonitril der Formel (I). Ein anderer Aspekt der Erfindung ist die Verwendung des erfindungsgemäßen Verfahrens zur Herstellung von 4-Formyl-3-Methoxybenzonitril der Formel (I) zur Herstellung von Finerenone nach Formel (II). Ein anderer Aspekt der Erfindung ist ein Verfahren zur Herstellung von Finernone (II) unter Verewndung des Verfahrens zur Herstellung von 4-Formyl-3-methoxybenzonitril der Formel (I). Ein anderer Aspekt der Erfindung ist die Verwendung von 4-Methyl-3-Methoxybenzonitril der Formel (III) oder 3-Methoxy-4-(dibromomethyl)benzonitril (IV) zur Herstellung von 4-Formyl-3-Methoxybenzonitril der Formel (I). Ein anderer Aspekt der Erfindung ist die Verwendung von 4-Methyl-3-Methoxybenzonitril der Formel (III) oder 3-Methoxy-4-(dibromomethyl)benzonitril (IV) zur Herstellung von Finerenone nach Formel (II).

## Beschreibung

Die vorliegende Erfindung betrifft ein Verfahren zur Herstellung von 4-Formyl-3-methoxybenzonitril der Formel (I) 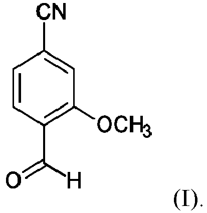 4-Formyl-3-methoxybenzonitril (I) ist ein zentraler Baustein bei der Herstellung von Finerenone, (4S)-4-(4-Cyano-2-methoxyphenyl)-5-ethoxy-2,8-dimethyl-1,4-dihydro-1,6-naphthyridin-3-carbox-amid, der Formel (II)

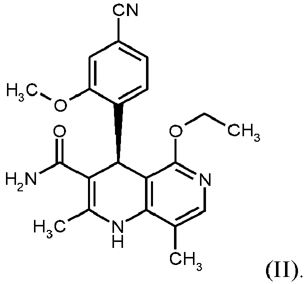

Die Verbindung der Formel (II) wirkt als nicht steroidaler Antagonist des Mineralcorticoid Rezeptors und wird zur Prophylaxe und/oder Behandlung von kardiovasculären und renalen Erkrankungen eingesetzt. Die CAS Nummer von Finerenone ist CAS 1050477-31-0. Die Verbindung der Formel (I) und dessen Herstellungsprozess sind beispielsweise in WO 2008/104306 A1, ChemMedChem 2012, 7, 1385 und EP 3174875 B1 beschrieben. Der Aldehyd nach der Formel (I) ist literaturbekannt, beispielsweise in EP 3174875 B1. In der EP 3174875 B1 ist die Herstellung der Verbindung der Formel (I) wie folgt beschreiben:

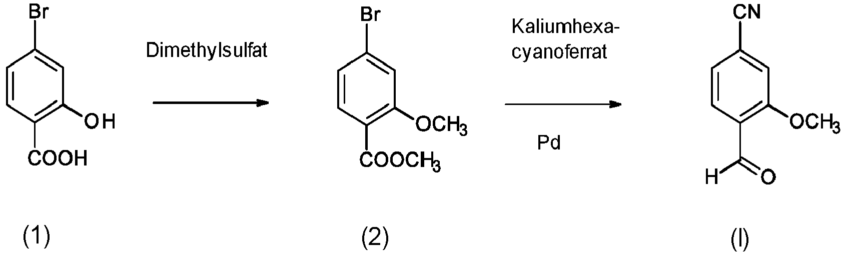

In der EP 3174875 B1 erfolgt die erste Stufe von der Verbindung der Formel (1) zu der Verbindung der Formel (2) in Dimethylsulfat. Die Einführung der Nitrilgruppe in der Verbindung nach Formel (I) in der zweiten Stufe erfolgt mittels Palladium-Katalyse/Kaliumhexacyanoferrat. Die Vorgehensweise birgt unterschiedliche Nachteile: Dimethylsulfat wird als hoch giftig und kanzerogen eingestuft. Der Einsatz vom hoch giftigen und kanzerogenen Dimethylsulfat macht ein Up-Scaling dieser Stufe im großen Maßstab schwierig. Es müssen zahlreiche Sicherheitsmaßnahmen eingehalten werden, um einen sicheren Umgang zu gewährleisten. Auch das Ausgangsmaterial Bromsalicylsäure der Formel (1) ist teuer. Ferner müssen die zu entsorgenden Abfälle gesondert aufgearbeitet werden, um sicher zu stellen, dass das Dimethylsulfat vollständig vernichtet ist, was zu insgesamt erhöhten Kosten führt.

Weitere Synthesen für die Verbindung der Formel (I) sind beispielsweise in WO2007140894 A1, WO2001000587 A1, WO2006060461 A1, US20130072468 A1, und in Banerjee, Abhisek; et al. Bioorganic & Medicinal Chemistry Letters (2012), 22(9), 3223-3228 beschrieben. Alle in diesen Literaturstellen beschriebenen Verfahren sind für ein industrielles Upscaling nicht geeignet, da die verwendeten Reagenzien zum einen ein Gefahrenpotential beinhalten, und/oder der entstehende Abfall (beispielsweise Mangandioxid) schwierig zu beseitigen ist.

Die Herstellung von Aldehyd (I) ist beispielsweise in Patrick et al, Journal of Medicinal. Chemistry 2007, 50, 2468-2485, EP1719767 A1 und CN102020587 A beschrieben:

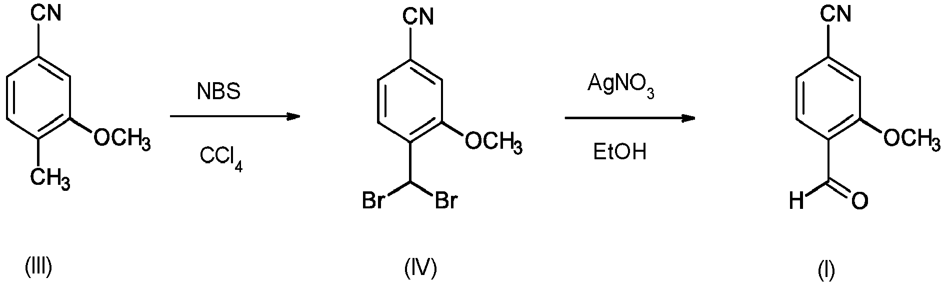

In EP'767 wird ausgehend vom 4-Cyano-2-methoxytoluol der Formel (III) [Verbindung 64 in EP'767] mit N-Bromosuccinimid (NBS) in Tetrachlorkohlenstoff (CCl₄) 3-Methoxy-4-(dibromomethyl)benzonitril der Formel (IV) [Verbindung 65 in EP'767] hergestellt, welches in Ethanol mit 2,46 Äquivalenten Silbernitrat zum Aldehyd der Formel (I) [Verbindung 44f in EP'767] umgesetzt wird. Außerdem wird das Zwischenprodukt Dibromid der Formel (IV) (3-Methoxy-4-(dibromomethyl)benzonitril) noch mit einer teuren Chromatographie-Stufe aufgereinigt. Diese in der Literatur beschriebene Synthesen sowie das in der Forschungs-Synthese beschriebene Verfahren sind für ein Up-Scaling in den Multi-Tonnen-Maßstab nicht geeignet. Insbesondere trifft dies unter Berücksichtigung der Funktion des Silbers als HBr-Fänger zu: Die Verseifung von benzylischen Dibromiden unter wasserfreien Bedingungen ist an sich bekannt (DMSO 100°C: Li, Wei; et al Tetrahedron Letters (2004), 45(5), 1071-1074). Umsetzungen unter den in Li et al. (2004) beschriebenen Bedingungen, führt zu vielen Nebenreaktionen aufgrund von Zersetzungen, die zu stark verunreinigten Produkten führen (vergleiche auch: DMF: 120°C: Wang, Yongfei; et al Synthesis (2011), (2), 287-291). Unter wasserfreien Bedingungen beobachtet man die Spaltung des Methylethers und auch Anlagerung von HBr an die Nitrilgruppe, sowie auch Kembromierungs-Nebenprodukte.

In CN102020587 A wird die Hydrolyse mit Dimethylsulfoxid (als Lösungsmittel) durchgeführt, was sich sehr schwer aus dem entstehenden Reaktionsgemisch abtrennen lässt. Es entsteht außerdem eine starke Geruchsbelastung durch entstehendes Dimethylsulfid, die besondere Vorkehrungen, wie Oxidationswäscher, erfordern. Der Aldehyd der Formel (I), der durch Extraktion aus Essigester erhalten wird, wird lediglich zur Trockne eingedampft. Ein Kristallisation- und Isolierungsverfahren ist nirgendwo offenbart.

Für einen Einsatz in der pharmazeutischen Industrie ist die Qualität der den oben diskutierten Synthesen anfallenden Produkte nicht ausreichend. Da es sich beim Aldehyd der Formel (I) um ein sogenanntes "Regulatory Starting Material" handelt, sind besondere Anforderungen an die Reinheit (> 99 %) dieser Substanz zu stellen. Das heißt, dass neben der eigentlichen Synthese noch ein Downstream Kristallisations-Prozess gehört, der für eine hohe Reinheit und Reproduzierbarkeit sorgt. Da alle diese Anforderungen im Stand der Technik nicht beschrieben sind, bestand ein hoher Bedarf nach einer neuen effizienteren und ökonomisch günstigeren Synthese, die den Aldehyd der Formel (I) in sehr hoher Reinheit zur Verwendung in einer für die pharmazeutische Industrie geeignete Synthese liefert.

Es ist gelungen durch ein neues Verfahren den Anforderungen an ein skalierbares Verfahren hinsichtlich Umweltverträglichkeit der Materialien und Kosteneffizienz gerecht zu werden Das neue Verfahren lässt sich grundsätzlich wie nachfolgend beschreiben:

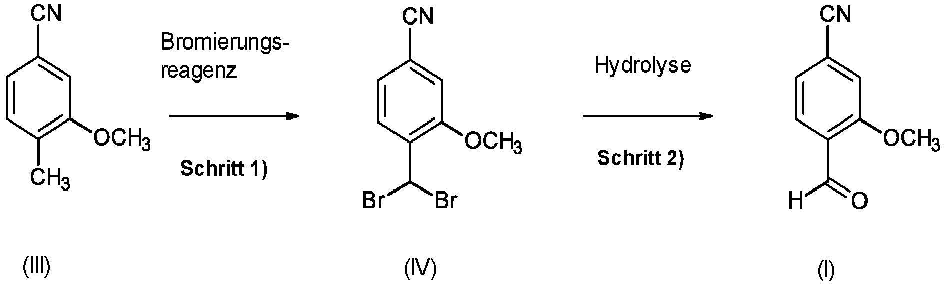

Die Erfindung betrifft ein Verfahren zur Herstellung von 4-Formyl-3-Methoxybenzonitril der Formel (I), 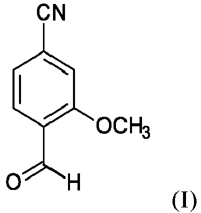 umfassend die Schritte
Schritt 1) Umsetzen von 4-Methyl-3-Methoxybenzonitril der Formel (III) 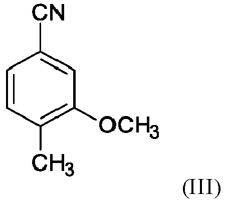 mit einem Bromierungsreagenz, wobei 3-Methoxy-4-(dibromomethyl)benzonitril der Formel (IV) 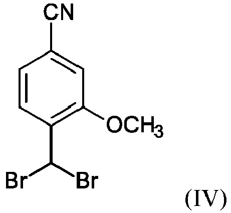 erhalten wird,
Schritt 2) Hydrolyse von 3-Methoxy-4-(dibromomethylbenzonitril) der Formel (IV) 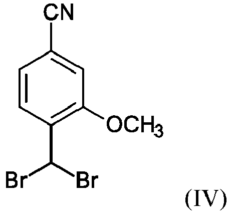 zu der Verbindung der Formel (I)

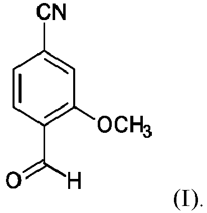

Die Begriffe "4-Formyl-3-methoxybenzonitril" und "Verbindung nach Formel (I)" sind Synonyme. Die Begriffe "Finerenone", Verbindung der Formel (II) und (4S)-4-(4-Cyano-2-methoxyphenyl)-5-ethoxy-2,8-dimethyl-1,4-dihydro-1,6-naphthyridin-3-carbox-amid sind Synonyme. Die Begriffe "4-Methyl-3-Methoxybenzonitril" und "Verbindung nach Formel (III)" sind Synonyme. Die Begriffe "3-Methoxy-4-(dibromomethyl)benzonitril" und "Verbindung nach Formel (IV)" sind Synonyme.

Das erfindungsgemäße Verfahren hat darüber hinaus den großen Vorteil, dass das Dibromid der Formel (IV) nicht isoliert werden muss, sondern direkt in darauffolgenden Schritt eingesetzt werden kann. Diesen direkten Einsatz ohne weitere Isolierung nennt der Fachmann auch "teleskopieren". Somit kann das Dibromid (II) direkt in den darauffolgenden Schritt teleskopiert werden. Beispielsweise spart dies nicht nur weitere Schritte, Material, sondern auch den Einsatz aufwendiger und kostenintensiver Isolierapparate. Der Zusatz von Wasser bei der Hydrolyse des Dibromids der Formel (IV) führt zu sehr reinem Produkt der Formel (I). Ferner sind kaum Nebenprodukte oder in nur sehr geringen Mengen vorhanden. Das erfindungsgemäße Verfahren liefert den Aldehyd der Formel (I) in sehr hoher chemischer Reinheit (> 99 %). Dies ermöglicht es, dieses Ausgangsmaterial für die Synthese eines Arzneistoff verwendet werden kann. In einer Ausführungsform wird die Verbindung der Formel (I) für die Herstellung der Verbindung nach Formel (II) verwendet.

In Schritt 1) des erfindungsgemäßen Verfahrens wird 4-Methyl-3-Methoxy-benzonitril der Formel (III) 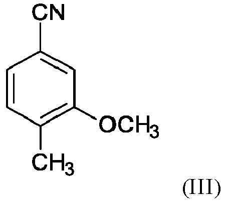 mit einem Bromierungsreagenz umgesetzt, wobei 3-Methoxy-4-(dibromomethylbenzonitril) der Formel (IV) 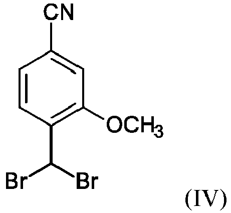 erhalten wird.

3-Methoxy-4-Methylbenzonitrilder Formel (III) ist kommerziell erhältlich. Die CAS Nummer ist CAS 3556-60-3. Die Herstellung der Verbindung der Formel (III) ist literaturbekannt (siehe beispielsweise WO2007139992 A2, Example 1).

Es ist auch möglich weitere auf dem Markt erhältliche Bromierungsreagenzien in Schritt 1) zu verwenden. Grundsätzlich sind bromhaltige Amide, bromhaltige Imide oder Mischungen davon geeignet, bei denen das Brom am Stickstoff-Atom sitzt. In einer Ausführungsform ist das Bromierungsreagenz ausgewählt aus N-Bromsuccinimid, 1,3-Dibrom-5,5-hydantoin, Brom und Mischungen davon. In einer Ausführungsform ist das Bromierungsreagenz ausgewählt aus N-Bromsuccinimid, 1,3-Dibrom-5,5-hydantoin und Brom. In einer Ausführungsform ist das Bromierungsreagenz 1,3-Dibrom-5,5-hydantoin. In einer Ausführungsform werden in Schritt 1) 2 bis 3 Äquivalente N-Bromsucciniimid, bezogen auf das Ausgangsmaterial (III), eingesetzt. In einer Ausführungsform werden in Schritt 1) 2,0 -2,5 Äquivalente N-Bromsucciniimid, bezogen auf das Ausgangsmaterial (III), eingesetzt. In einer Ausführungsform werden in Schritt 1) 2,1-2,2 Äquivalente N-Bromsucciniimid, bezogen auf das Ausgangsmaterial (III), eingesetzt. In einer Ausführungsform werden in Schritt 1) 1,0 bis 1,5 Äquivalente 1,3-Dibrom-5,5-hydantoin, bezogen auf das Ausgangsmaterial (III), eingesetzt. In einer Ausführungsform werden in Schritt 1) 1,1 bis 1,3 Äquivalente 1,3-Dibrom-5,5-hydantoin, bezogen auf das Ausgangsmaterial (III), eingesetzt. In einer Ausführungsform werden in Schritt 1) 1,1 bis 1,2 Äquivalente 1,3-Dibrom-5,5-hydantoin, bezogen auf das Ausgangsmaterial (III), eingesetzt. In einer Ausführungsform von Schritt 1) wird Brom (Br₂) als Bromierungsreagenz eingesetzt. In einer Ausführungsform von Schritt 1) wird Brom als Bromierungsreagenz in einer Menge von 1 bis 3 Äquivalenten, bezogen auf das Ausgangsmaterial (III), eingesetzt. In einer Ausführungsform von Schritt 1) wird Brom als Bromierungsreagenz in einer Menge von 2,0 bis 2,4 Äquivalenten, bezogen auf das Ausgangsmaterial (III), eingesetzt. In einer Ausführungsform von Schritt 1) wird Brom als Bromierungsreagenz eingesetzt und bestrahlt. In einer Ausführungsform von Schritt 1) wird Brom als Bromierungsreagenz eingesetzt und mit einer Tauchlampe bestrahlt. Beim Einsatz der Tauchlampe erfolgt der Radikalstart durch das Licht.

In einer Ausführungsform wird in Schritt 1) ein Lösungsmittel eingesetzt. Der Begriff "Lösungsmittel" umfasst auch Lösungsmittelgemische. In einer Ausführungsform ist das Lösungsmittel in Schritt 1) ein chloriertes Lösungsmittel. In einer Ausführungsform ist das Lösungsmittel in Schritt 1) ausgewählt aus Chloroform, Chlorbenzol, Dichlorbenzol und Mischungen davon. In einer Ausführungsform ist das Lösungsmittel in Schritt 1) ausgewählt aus Chlorbenzol, Chloroform und Mischungen davon.

In einer Ausführungsform wird die Bromierung in Schritt 1) bei Temperaturen von 50 bis 120°C durchgeführt. In einer Ausführungsform wird die Bromierung in Schritt 1) bei Temperaturen von 80 bis 100°C durchgeführt. In einer Ausführungsform wird die Bromierung in Schritt 1) bei Temperaturen von 85 bis 95°C durchgeführt.

In einer Ausführungsform von Schritt 1) wird eine 5 bis 15-fache Menge des Lösungsmittels eingesetzt, wobei die Menge des Lösungsmittels die Volumenmenge in Mililiter (mL), bezogen auf 1 Gramm (g) eingesetztes Ausgangsmaterial, ist. Beispielsweise wird bei einem Einsatz der 5-fachen Menge des Lösungsmittels 5 mL des Lösungsmittels für 1 g der Verbindung nach Formel (III) eingesetzt. In einer Ausführungsform von Schritt 1) wird eine 8-10 fache Menge des Lösungsmittels eingesetzt, wobei die Menge des Lösungsmittels die Volumenmenge in mL, bezogen auf 1 g eingesetztes Ausgangsmaterial, ist.

In einer Ausführungsform von Schritt 1) wird ein Radikalstarter eingesetzt werden. Radikalstarter sind in der Literatur hinreichend bekannt. Sie können zum kontrollierten Starten der Reaktion eingesetzt werden. In einer Ausführungsform ist der Radikalstarter ausgewählt aus Dibenzoylperoxid und Azobisisobutyronitril. In einer Ausführungsform von Schritt 1) ist der Radikalstarter Dibenzoylperoxid In einer Ausführungsform von Schritt 1) ist der Radikalstarter Azobisisobutyronitril. Es ist möglich auch andere auf dem Markt erhältliche Radiaklastarter einzusetzten. Es können reaktive Peroxide, reaktive Azoverbindungen oder Licht eingesetzt werden.

In einer Ausführungsform von Schritt 1) wird der Radikalstarter in einer katalytischen Menge eingesetzt. In einer Ausführungsform von Schritt 1) wird der Radikalstarter in einer Menge von 0,03 bis 0,15 Äuqivalenten, bezogen auf das Ausgangsmaterial (III), eingesetzt. In einer Ausführungsform von Schritt 1) wird der Radikalstarter in einer Menge von 0,05 bis 0,1 Äquivalenten, bezogen auf das Ausgangsmaterial (III), eingesetzt. In einer Ausführungsform von Schritt 1) wird der Radikalstarter in einer Menge von 0,05 Äquivalenten, bezogen auf das Ausgangsmaterial (III), eingesetzt. Zusätzlich zu dem Radikalstarter kann eine kleine Menge elementares Brom zugesetzt werden. In einer Ausführungsform von Schritt 1) wird zusätzlich elementares Brom in einer Menge von 0,01 Äquivalenten, bezogen auf das Ausgangsmaterial (III), eingesetzt.

In einer Ausführungsform von Schritt 1) beträgt die Reaktionszeit 3 bis 8 Stunden. In einer Ausführungsform von Schritt 1) beträgt die Reaktionszeit 4 bis 5 Stunden.

Zur Aufarbeitung des Reaktionsgemisches oder des Produkts aus Schritt 1) kann mit Wasser und/oder einer Natriumsulfit oder Natriumhydrogensulfit oder Natriumthiosulfat-Lösung gewaschen, von ausgefallenen Feststoffen abfiltriert und das Lösungsmittel bis zur Rührbarkeitsgrenze abdestilliert werden.

In Schritt 2) des erfindungsgemäßen Verfahrens wird 3-Methoxy-4-(dibromomethylbenzonitril) der Formel (IV) 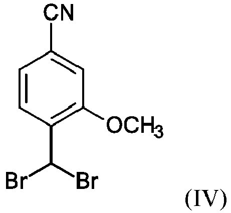 zu der Verbindung der Formel (I) 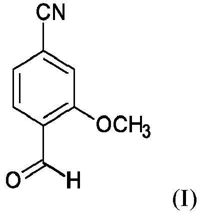 hydrolysiert.

Die Hydrolyse hat große Vorteile gegenüber denen im Stand der Technik beschriebenen Verfahren, s.o. (AgNO3: J. Med. Chem. 2007, 50, 2468-2485, EP1719767 A1).

In einer Ausführungsform wird das in Schritt 1) erhaltene Produkt, welches die Verbindung der Formel (IV) umfasst, direkt in den nächsten Schritt 2) eingesetzt. In einer Ausführungsform wird aus dem in Schritt 1) erhaltenen Produkt die Verbindung der Formel (IV) isoliert. Die Isolierung kann beispielsweise durch Kristallisation erfolgen. Die isolierte Verbindung der Formel (IV) kann dann in Schritt 2) zu der Verbindung der Formel (I) hydrolysiert werden. In einer Ausführungsform von Schritt 2) ist die Hydrolyse eine wässrige Hydrolyse.

In einer Ausführungsform von Schritt 2) wird die Hydrolyse des Dibromides der Formel (IV) in einem Lösungsmittel durchführt. Der Begriff "Lösungsmittel" umfasst auch Lösungsmittelgemische. In einer Ausführungsform von Schritt 2) ist Lösungsmittel ausgewählt aus Wasser, Dimethylformamid, Dimethylacetamid, N-Methylpyrolidon (NMP), Tetramethylharnstoff, Chlorbenzol, Dichlorbenzol und Mischungen davon. In einer Ausführungsform von Schritt 2) ist Lösungsmittel ausgewählt aus Wasser, Chlorbenzol, Chloroform, Dimethylformamid und Mischungen davon. In einer Ausführungsform von Schritt 2) ist Lösungsmittel ausgewählt aus Chlorbenzol, Chloroform, Dimethylformamid und Mischungen davon.

In einer Ausführungsform von Schritt 2) ist das Lösungsmittel ein Lösungsmittelgemisch umfassend Wasser und ein Lösungsmittel. In einer Ausführungsform von Schritt 2) ist das Lösungsmittel ein Lösungsmittelgemisch umfassend Wasser und ein Lösungsmittel ausgewählt aus Dimethylacetamid, N-Methylpyrolidon (NMP), Tetramethylharnstoff, Chlorbenzol, Dichlorbenzol und Mischungen davon. In einer Ausführungsform von Schritt 2) ist das Lösungsmittel ein Lösungsmittelgemisch umfassend Wasser und ein Lösungsmittel ausgewählt aus Chlorbenzol, Chloroform, Dimethylformamid und Mischungen davon. In einer Ausführungsform von Schritt 2) ist das Lösungsmittel ein Lösungsmittelgemisch bestehend aus Wasser und einem Lösungsmittel. In einer Ausführungsform von Schritt 2) ist das Lösungsmittel ein Lösungsmittelgemisch bestehend aus Wasser und ein Lösungsmittel ausgewählt aus Dimethylacetamid, N-Methylpyrolidon (NMP), Tetramethylharnstoff, Chlorbenzol, Dichlorbenzol und Mischungen davon. In einer Ausführungsform von Schritt 2) ist das Lösungsmittel ein Lösungsmittelgemisch bestehend aus Wasser und ein Lösungsmittel ausgewählt aus Chlorbenzol, Chloroform, Dimethylformamid und Mischungen davon. In einer Ausführungsform von Schritt 2) ist das Lösungsmittel ein Lösungsmittelgemisch bestehend aus Wasser und einem Lösungsmittel, wobei das Volumenverhältnis von Wasser zu Lösungsmittel ausgewählt ist aus 1:3, 1:2 und 1:1. In einer Ausführungsform von Schritt 2) ist das Lösungsmittel ein Lösungsmittelgemisch bestehend aus Dimethylformamid und Wasser. In einer Ausführungsform von Schritt 2) ist das Lösungsmittel ein Lösungsmittelgemisch bestehend aus Dimethylformamid und Wasser, wobei das Volumenverhältnis von Dimethylformamid zu Wasser 1 bis 1,5 :1 beträgt. In einer Ausführungsform von Schritt 2) ist das Lösungsmittel ein Lösungsmittelgemisch bestehend aus Chlorbenzol und Wasser. In einer Ausführungsform von Schritt 2) ist das Lösungsmittel ein Lösungsmittelgemisch bestehend aus Chlorbenzol und Wasser, wobei das Volumenverhältnis von Chlorbenzol zu Wasser 1,5 : 1 beträgt. In einer Ausführungsform von Schritt 2) ist das Lösungsmittel ein Lösungsmittelgemisch bestehend aus Chloroform und Wasser. In einer Ausführungsform von Schritt 2) ist das Lösungsmittel ein Lösungsmittelgemisch bestehend aus Chloroform und Wasser. In einer Ausführungsform von Schritt 2) ist das Lösungsmittel ein Lösungsmittelgemisch bestehend aus Chloroform und Wasser, wobei das Volumenverhältnis von Chlorbenzol zu Wasser 1,5 :1 beträgt.

Der Schritt 2) kann in einer homogenen Phase oder zweiphasigen Umsetzung erfolgen. Bei einer zweiphasigen Umsetzung kann intensiv gerührt werden, damit eine gute Durchmischung erfolgen kann. Zur praktischen Durchführung des Schritts 2) kann man gegebenenfalls erst einen Überschuss des Lösungsmittels zugeben und auf das Zielvolumen abdestilliert werden. Anschließend kann Wasser mit sauren oder basischen Zusätzen zugegeben werden.

In einer Ausführungsform von Schritt 2) wird dem Reaktionsgemisch ein saurer Zusatz beigefügt. In einer Ausführungsform von Schritt 2) wird dem Reaktionsgemisch ein saurer Zusatz ausgewählt aus Salzsäure, Bromwasserstoffsäure, Schwefelsäure, Phosphorsäure, Ameisensäure, Essigsäure und Mischung davon beigefügt.

In einer Ausführungsform von Schritt 2) wird dem Reaktionsgemisch ein basischer Zusatz ausgewählt aus Natriumhydroxid, Kaliumhydroxid, Calciumhydroxid, Natriumcarbonat, Natriumhydrogencarbonat, Kaliumcarbonat, Kaliumhydrogencarbonat, Natriumphosphat, Kaliumphosphat, Pyridin, Triethylamin, Ammoniak und Mischung davon beigefügt. Es können auch In einer Ausführungsform von Schritt 2) wird dem Reaktionsgemisch ein basischer Zusatz ausgewählt aus Natriumhydroxid, Natriumcarbonat, Natriumhydrogencarbonat und Mischungen davon beigefügt.

In Schritt 2) können die unter Schritt 1) oder Schritt 2) genannten Lösungsmittel oder Lösungsmittelgemische eingesetzt werden. Dies gilt auch andersherum.

In einer Ausführungsform von Schritt 2) erfolgt die Hydrolyse bei Temperaturen von 80 bis 100 °C. In einer Ausführungsform von Schritt 2) erfolgt die Hydrolyse bei Temperaturen von 85 bis 95 °C. In einer Ausführungsform von Schritt 2) erfolgt die Hydrolyse bei 90 °C.

In einer Ausführungsform von Schritt 2) beträgt die Reaktionszeit 2 bis 6 Stunden. In einer Ausführungsform von Schritt 2) beträgt die Reaktionszeit 3 bis 4 Stunden. In einer Ausführungsform von Schritt 2) beträgt die Reaktionszeit 4 Stunden.

Zur Aufarbeitung des in Schritt 2) erhaltenen Produkts kann Wasser und/oder eine anorganische oder organische Base zugegeben werden, wie beispielsweise Natriumhydroxid, Kaliumhydroxid, Calciumhydroxid, Natriumcarbonat, Natriumhydrogencarbonat, Kaliumcarbonat, Kaliumhydrogencarbonat, Natriumphosphat, Kaliumphosphat, Pyridin, Triethylamin, Ammoniak. In einer Ausführungsform wird Natriumcarbonat und/oder Natriumhydrogencarbonat verwendet. Es kann gegebenenfalls auch mit Natriumsulfit-, Natriumhydrogensulfit- oder Natriumthiosulfat-Lösung nachgewaschen werden. Man kann die Base in gelöster Form in Wasser dazugeben. Gegebenenfalls kann der pH-Wert durch Zugabe einer Säure eingestellt werden. In einer Ausführungsform wird er pH auf 5-8 eingestellt. In einer Ausführungsform wird der pH auf 7 eingestellt. In einer Ausführungsform ist die Säure Salzsäure.

Anschließend an den Schritt 2) kann die Verbindung extrahiert werden. In einer Ausführungsform umfasst das Verfahren Schritt 1), Schritt 2) und einen Extraktionsschritt. In einer Ausführungsform des Extrahierungsschritts kann die Verbindung der Formel (I) mit einem nicht mit Wasser mischbaren Lösungsmittel extrahiert werden. In einer Ausführungsform ist das Lösungsmittel ausgewählt aus Essigester, Methylisobutylketon (MIBK), Methylethylketon (MEK), Methyl-tert-Butylether (MTBE), Isopropylacetat, THF, 2-Methyl-THF Dichlormethan und Mischungen davon. In einer Ausführungsform ist das Lösungsmittel ausgewählt aus MIBK, Essigester, Chlorbenzol und Mischungen davon.

Anschließend an Schritt 2) kann ein weiter Aufreinigungsschritt vorgenommen werden. Dieser kann nach Schritt 2) oder dem Extrahierungsschritt vorgenommen werden. In einer Ausführungsform ist der Aufreinigungsschritt ein Kristallisationsschritt.

In einer Ausführungsform umfasst das Verfahren Schritt 1), Schritt 2), einen Extraktionsschritt und einen Aufreinigungsschritt. In einer Ausführungsform umfasst das Verfahren Schritt 1), Schritt 2) und einen Aufreinigungsschritt. In einer Ausführungsform umfasst das Verfahren Schritt 1), Schritt 2), einen Extraktionsschritt und einen Kristallisationsschritt. In einer Ausführungsform umfasst das Verfahren Schritt 1), Schritt 2) und einen Kristallisationsschritt. In einer Ausführungsform des Kristallisationsschritts wird ein Lösungsmittel ausgewählt aus Essigester, Methyl-tert-butylether (MTBE), Isopropylacetat, Isobutylmethylketon (MIBK), Hexan, Cyclohexan, Methylcyclohexan, Heptan, Aceton, Chlorbenzol, Toluol, Dichlormethan und Mischungen davon eingesetzt. In einer Ausführungsform des Kristallisationsschritts wird ein Lösungsmittel ausgewählt aus Essigester, Methyl-tert-butylether (MTBE), Isopropylacetat, Isobutylmethylketon(MIBK) und Mischungen davon eingesetzt. des Kristallisationsschritts wird ein Lösungsmittel ausgewählt aus Hexan, Cyclohexan, Methylcyclohexan, Heptan, Aceton, Essigester, Chlorbenzol, Isobutylmethylketon (MIBK), Methyl-tert-Butylether (MTBE), Toluol und Mischungen davon eingesetzt. In einer Ausführungsform des Kristallisationsschritts wird ein Lösungsmittel ausgewählt aus Heptan, Chlorbenzol, Isobutylmethylketon (MIBK), Essigester, Dichlormethan oder Mischungen davon eingesetzt. In einer Ausführungsform des Kristallisationsschritts wird aus Essigester kristallisiert. In einer Ausführungsform des Kristallisationsschritts wird eines oder mehrere der unter Schritt 1) oder Schritt 2) genannten Lösungsmittel oder Lösungsmittelgemische eingesetzt werden.

In einer Ausführungsform des Kristallisationsschritts wird die Kristallisation bei Temperaturen von 20 bis -5 °C durchgeführt. In einer Ausführungsform des Kristallisationsschritts wird die Kristallisation bei Temperaturen von -10 bis -15 °C durchgeführt.

In einer Ausführungsform des Kristallisationsschritts wird eine stark konzentrierte, übersättigte Lösung, die die Verbindung nach Formel (I) enthält, in Essigester, Methyl-tert-butylether (MTBE), Isopropylacetat, Isobutylmethylketon oder Mischung davon bei tiefen Temperaturen von -20 bis -5°C oder -10 bis -15°C mit dem Aldehyd nach der Formel (I) angeimpft werden. In einer Variante dieser Ausführungsform wird mit ≤ 0,01 Äquivalente 1 des Aldehyds nach der Formel (I) angeimpft. Durch das Animpfen erfolgt eine kontrollierte Kristallisation beim Nachrühren.

Der auskristallisierte Aldehyd der Formel (I) kann weiterhin isoliert werden. In einer Ausführungsform kann die Isolierung durch Filtration erfolgen. In einer andern Ausführungsform kann dies mittels einer Zentrifuge erfolgen. Es sind aber weitere Isolierungsverfahren literaturbekannt und einsetzbar. Die isolierte Verbindung nach Formel (I) kann gegebenenfalls mit wenig kalten Lösungsmittel nachgewaschen werden. In einer Ausführungsform sind diese einsetzbaren Lösungsmittel ausgewählt aus Heptan und Chlorbenzol, Isobutylmethylketon (MIBK), Essigester, Dichlormethan und Mischungen davon.

In einem weiteren Schritt kann die Verbindung der Formel (I) nach einem oder mehreren der vorgenannten Schritte getrocknet werden. Die Trocknung Verbindung der Formel (I) kann bei 20 bis 55°C oder 45 bis 50°C erfolgen. In einer weiteren Ausführungsform der Trocknung wird im Vakuum gearbeitet. In einem Beispiel dieser Ausführungsform wird unter Verwendung von Stickstoff als Schleppgas gearbeitet. Es kann einem Druck von bei 20 bis 50 mbar gearbeitet werden.

In einer Ausführungsform umfasst das Verfahren ferner einen intermediären Aufreinigungsschritt der Verbindung nach Formel (I) nach Schritt 2). Der einer Ausführungsform umfasst der Aufreinigungsschritt die Schritte
(i) Umsetzung von 4-Formyl-3-Methoxybenzonitril der Formel (I) mit einer Bisulfitverbindung, wobei das Addukt nach Formel (V) 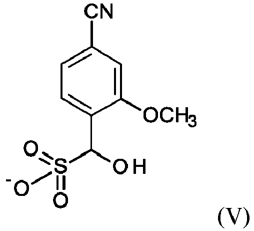 erhalten wird,
(ii) basische Spaltung des Addukts nach Formel (V), wobei 4-Formyl-3-Methoxybenzonitril der Formel (I) erhalten wird.

Die Verbindung der Formel (V) stellt ein Bisulfitaddukt dar. In einer Ausführungsform ist die Bisulfitverbindung in Schritt (i) ausgewählt aus NaHSO₃, KHSO₃ und Mischungen davon. In einer Ausführungsform von Schritt (i) werden die unter Schritt 2) genannten Lösungsmittel eingesetzt. Weitere Mischungen mit Alkoholen sind möglich. Beispiele für Alkohole sind Methanol, Ethanol, Isopropanol und Mischungen davon.

In Schritt (ii) wird die Verbindung der Formel (V) unter basischen Bedingungen wieder in den Aldehyd nach Formel (I) gespalten. Optional kann die Verbindung der Formel (V) erst isoliert und dann der basischen Spaltung unterworfen werden.

In einer Ausführungsform umfasst das Verfahren Schritt 1), Schritt 2), einen Extraktionsschritt und einen Aufreinigungsschritt umfassend die Schritte (1) und (ii). In einer Ausführungsform umfasst das Verfahren Schritt 1), Schritt 2) und einen Aufreinigungsschritt umfassend die Schritte (1) und (ii). In einer Ausführungsform umfasst das Verfahren Schritt 1), Schritt 2), einen Extraktionsschritt, einen Kristallisationsschritt und einen Aufreinigungsschritt umfassend die Schritte (1) und (ii). In einer Ausführungsform umfasst das Verfahren Schritt 1), Schritt 2), einen Kristallisationsschritt und einen Aufreinigungsschritt umfassend die Schritte (1) und (ii).

Zur intermediären Aufreinigung des Aldehyds nach der Formel (I), umfassend die Schritte (i) und (ii), kann auch eine Umsetzung zu einem Bisulfit Addukt (V) mit NaHSO₃ oder KHSO₃ erfolgen, wobei sich jeweils das Addukt nach Formel (Va) oder (Vb) bildet:

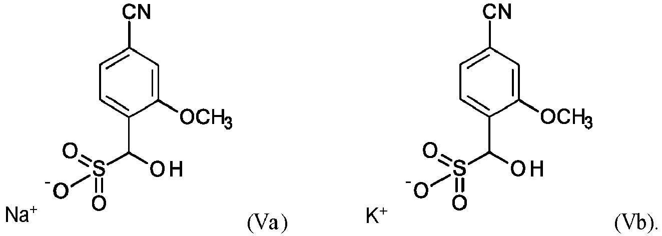

Beispielsweise wird der Einsatz der Bisulfite bei Kissane, Marie G.; et al. Tetrahedron Letters (2013), 54(48), 6587-6591 beschrieben. Die Herstellung des Bisulfit Addukts nach Formel (V) wird in Wasser in Kombination mit dem jeweils für die in Schritt 2) verwendeten Lösungsmittels durchgeführt. Dies kann auch zweiphasig erfolgen. In manchen Fällen wird das organische Lösungsmittel kontinuierlich abdestilliert. Das Bisulfit-Addukt nach Formel (V) wird isoliert und anschließend unter basischen Bedingungen wieder in den Aldehyd nach Formel (I) gespalten. Als Basen hierzu sind Natriumhydroxid, Natriumkarbonat, Natriumhydrogencarbonat, Natriumphosphat, sowie die jeweiligen Kalium-Analoga geeignet. Auch die Basen, die bereits unter Schritt 1) oder Schritt 2) genannt wurden, können eingesetzt werden. Nach Spaltung wird der Aldehyd nach Formel (I) in ein organisches Lösungsmittel extrahiert, bevorzugt Essigester, und dann der Kristallisation-Prozess durchgeführt, hierzu kann gegebenenfalls auf ein anderes Lösungsmittel umdestilliert werden. Unter "umdestillieren" wird auch ein sogenannter Lösungsmitteltausch oder sogenannter "solvent switch" verstanden.

Ein weiterer Aspekt der Erfindung ist ein Verfahren zur Herstellung von Finerenone nach Formel (II), 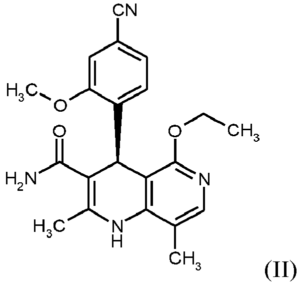 wobei das Verfahren die folgenden Schritte 1) und 2) umfasst:

### Schritt 1) Umsetzen von 4-Methyl-3-Methoxybenzonitril der Formel (III)

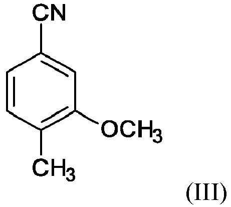

mit einen Bromierungsreagenz, wobei 3-Methoxy-4-(dibromomethyl)benzonitril der Formel (IV) 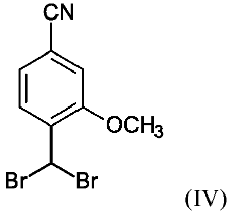 erhalten wird,

### Schritt 2) Hydrolyse von 3-Methoxy-4-(dibromomethylbenzonitril) der Formel (IV)

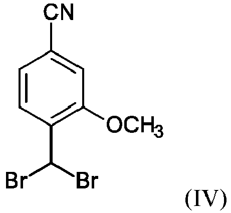

zu der Verbindung der Formel (I)

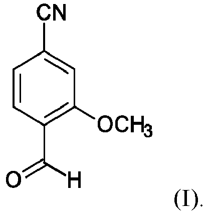

Ausführungsformen von Schritt 1) und Schritt 2) wurden bereits weiter oben beschrieben.

In einer Ausführungsform umfasst das Verfahren zur Herstellung von Finerenone (II) die Schritte 1), 2) und 3):

### Schritt 3) Umsetzen der Verbindung nach Formel (I)

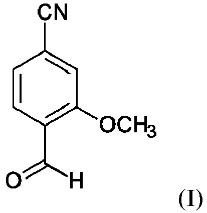

mit der Verbindung der Formel (VII) 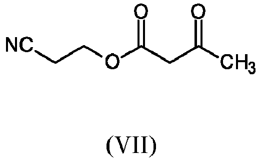 wobei die Verbindungen (VIIIa), (VIIIb) 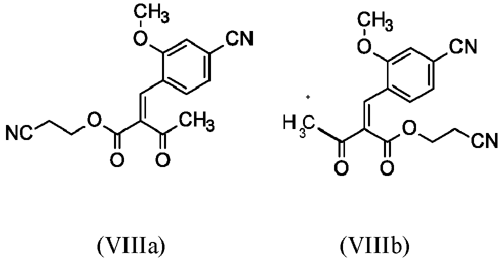 erhalten werden.

In einer Ausführungsform von Schritt 3) wird ein Lösungsmittel eingesetzt. In einer Ausführungsform von Schritt 3) wird als Lösungsmittel ein sekundärer Alkohol eingesetzt. In einer Ausführungsform von Schritt 3) wird ein Lösungsmittel ausgewählt aus Isopropanol, Isobutanol, 2-Amylalkohol, Cyclohexanol und Mischungen davon eingesetzt. In einer Ausführungsform von Schritt 3) wird als Lösungsmittel Isopropanol eingesetzt.

In einer Ausführungsform von Schritt 3) wird das Lösungsmittel in einer 3 bis 9-fachen Menge eingesetzt, wobei die Menge des Lösungsmittels die Volumenmenge in Mililiter (mL), bezogen auf 1 Gramm (g) eingesetztes Ausgangsmaterial (I), ist. In einer Ausführungsform von Schritt 3) wird das Lösungsmittel in einer 3 bis 8-fachen Menge eingesetzt, wobei die Menge des Lösungsmittels die Volumenmenge in Mililiter (mL), bezogen auf 1 Gramm (g) eingesetztes Ausgangsmaterial (I), ist. In einer Ausführungsform von Schritt 3) wird das Lösungsmittel in einer 5 bis 7-fachen Menge eingesetzt, wobei die Menge des Lösungsmittels die Volumenmenge in Mililiter (mL), bezogen auf 1 Gramm (g) eingesetztes Ausgangsmaterial (I), ist. In einer Ausführungsform von Schritt 3) wird als Lösungsmittel Isopropanol in einer 3 bis 9-fachen Menge eingesetzt, wobei die Menge des Lösungsmittels die Volumenmenge in Mililiter (mL), bezogen auf 1 Gramm (g) eingesetztes Ausgangsmaterial (I), ist. In einer Ausführungsform von Schritt 3) wird als Lösungsmittel Isopropanol in einer 3 bis 8-fachen Menge eingesetzt, wobei die Menge des Lösungsmittels die Volumenmenge in Mililiter (mL), bezogen auf 1 Gramm (g) eingesetztes Ausgangsmaterial (I), ist.

In einer Ausführungsform von Schritt 3) wird Isopropanol in einer 3 bis 8-fachen Menge und 0,05 bis 0,10 Äquivalente Piperidin, jeweils bezogen auf das Ausgangsmaterial (I), eingesetzt. In einer Ausführungsform von Schritt 3) wird Isopropanol in einer 3 bis 8-fachen Menge, 0,05-0,10 Äquivalente Piperidin und 0,05 bis 0,10 Äquivalente Eisessig, jeweils bezogen auf das Ausgangsmaterial (I), eingesetzt. In einer AusführungsformvonSchritt3) wirdIsopropanolineiner3 bis8-fachen Menge, 0,05-0,10 Äquivalente Piperidin und 0,05 bis 0,10 Äquivalente Eisessig gelöst, jeweils bezogen auf das Ausgangsmaterial (I), bei 30 °C eingesetzt. In einer Ausführungsform von Schritt 3) werden 1 bis 2 Äquivalente der Verbindung der Formel (VII), bezogen auf die Ausgangsverbindung (I), eingesetzt. In einer Ausführungsform von Schritt 3) werden 1 bis 1,5 Äquivalente der Verbindung der Formel (VII), bezogen auf die Ausgangsverbindung (I), eingesetzt. In einer Ausführungsform von Schritt 3) werden 1,2 bis 1,4 Äquivalente der Verbindung der Formel (VII), bezogen auf die Ausgangsverbindung (I), eingesetzt.

In einer Ausführungsform umfasst das Verfahren zur Herstellung von Finerenone (II) die Schritte 1), 2), 3) und 4)

### Schritt 4) Umsetzen der Verbindungen der Formeln (VIIIa), (VIIIb)

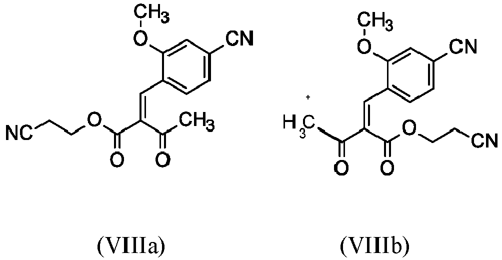

mit Amino-Methyl-Pyridonder Formel (IX) 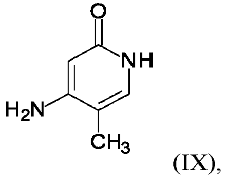 wobei die Verbindung der Formel (X) 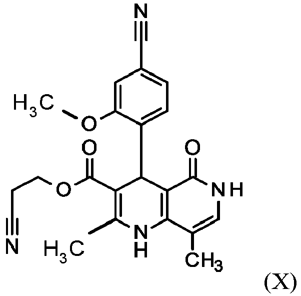 erhalten wird.

In einer Ausführungsform von Schritt 4) werden 0,2 bis 2,2 Äquivalente der Verbindung der Formel (IX), bezogen auf die Ausgangsverbindung (VIIIa, VIIIb), eingesetzt. Wenn hier von "bezogen auf die Ausgangsverbindung (VIIIa, VIIIb)" die Rede ist, dann wird auf die Gesamtmenge beider Verbindungen (VIIIa) und (VIIIb) Bezug genommen. In einer Ausführungsform von Schritt 4) werden 0,5 bis 1,5 Äquivalente der Verbindung der Formel (IX), bezogen auf die Ausgangsverbindung (VIIIa, VIIIb), eingesetzt. In einer Ausführungsform von Schritt 4) werden 0,5 bis 1,5 Äquivalente der Verbindung der Formel (IX), bezogen auf die Ausgangsverbindung (VIIIa, VIIIb), eingesetzt. In einer Ausführungsform von Schritt 4) werden 0,7 bis 1,2 Äquivalente der Verbindung der Formel (IX), bezogen auf die Ausgangsverbindung (VIIIa, VIIIb), eingesetzt. In einer Ausführungsform von Schritt 4) werden 0,8 bis 1 Äquivalente der Verbindung der Formel (IX), bezogen auf die Ausgangsverbindung (VIIIa, VIIIb), eingesetzt.

In einer Ausführungsform von Schritt 4) wird ein Lösungsmittel eingesetzt. In einer Ausführungsform von Schritt 4) wird als Lösungsmittel ein sekundärer Alkohol eingesetzt. In einer Ausführungsform von Schritt 4) wird ein Lösungsmittel ausgewählt aus Isopropanol, Isobutanol, 2-Amylalkohol, Cyclohexanol und Mischungen davon eingesetzt. In einer Ausführungsform von Schritt 3) wird als Lösungsmittel Isopropanol eingesetzt.

In einer Ausführungsform von Schritt 4) wird das Lösungsmittel in einer 3 bis 120-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. Wenn hier von "bezogen auf 1 Gramm (g) eingesetztes Ausgangsmaterial (VIIIa, VIIIb)" die Rede ist, dann wird auf die Gesamtmenge beider Verbindungen (VIIIa) und (VIIIb) Bezug genommen. Beispielsweise wird bei einem Einsatz der 3-fachen Menge des Lösungsmittels 3 mL des Lösungsmittels für 1 g (Gesamtgewicht) der Verbindungen nach Formeln (VIIIa) und (VIIIb) eingesetzt. In einer Ausführungsform von Schritt 4) wird das Lösungsmittel in einer 5 bis 18-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. In einer Ausführungsform von Schritt 4) wird das Lösungsmittel in einer 8 bis 15 -fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. In einer Ausführungsform von Schritt 4) wird das Lösungsmittel in einer 8,5 bis 14-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. In einer Ausführungsform von Schritt 4) wird das Lösungsmittel in einer 8 bis 10-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. In einer Ausführungsform von Schritt 4) wird als Lösungsmittel Isopropanol in einer 8 bis 15-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist. In einer Ausführungsform von Schritt 4) wird als Lösungsmittel Isopropanol in einer 8,5 bis 14-fachen Menge eingesetzt, wobei die Menge des Lösungsmittels die Volumenmenge in Mililiter (mL), bezogen auf 1 Gramm (g) eingesetztes Ausgangsmaterial (VIIIa, b), ist. In einer Ausführungsform von Schritt 4) wird als Lösungsmittel Isopropanol in einer 9 bis 10-fachen Menge eingesetzt, wobei die Menge des Lösungsmittels die Volumenmenge in Mililiter (mL), bezogen auf 1 Gramm (g) eingesetztes Ausgangsmaterial (VIIIa, VIIIb), ist.

In einer Ausführungsform von Schritt 4) wird der Schritt bei einer Temperatur von 70 bis 130°C durchgeführt. In einer Ausführungsform von Schritt 4) wird der Schritt bei einer Temperatur von 80 bis 120°C durchgeführt. In einer Ausführungsform von Schritt 4) wird der Schritt bei einer Temperatur von 90 bis 110°C durchgeführt. In einer Ausführungsform von Schritt 4) wird der Schritt bei einer Temperatur von 95 bis 105°C durchgeführt. In einer Ausführungsform von Schritt 4) wird der Schritt bei einer Temperatur von 100°C durchgeführt.

In einer Ausführungsform umfasst das Verfahren zur Herstellung von Finerenone (II) die Schritte 1), 2), 3), 4) und 5)

### Schritt 5) Umsetzen der Verbindung der Formel (X)

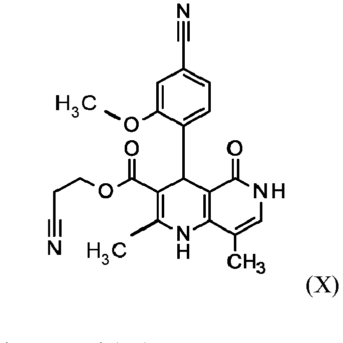

zu der Verbindung der Formel (XI)

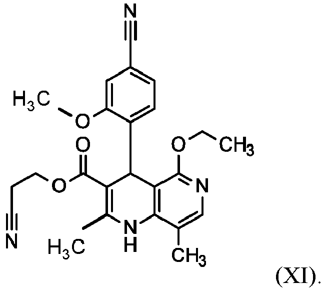

In einer Ausführungsform von Schritt 5) erfolgt die Umsetzung unter saurer Katalyse. In einer Ausführungsform von Schritt 5) erfolgt die Umsetzung unter Zugabe eines sauren Katalysators ausgewählt aus von Schwefelsäure, Orthoestern und Mischungen davon. In einer Ausführungsform von Schritt 5) erfolgt die Umsetzung unter Zugabe eines sauren Katalysators ausgewählt aus Schwefelsäure, Triethylorthoessigester und Mischungen davon. In einer Ausführungsform von Schritt 5) erfolgt die Umsetzung unter Zugabe von Triethylorthoessigester.

In einer Ausführungsform von Schritt 5) werden 2 bis 5 Äquivalente der Verbindung des sauren Katalysators, bezogen auf die Ausgangsverbindung (X), eingesetzt. In einer Ausführungsform von Schritt 5) werden 2 bis 4 Äquivalente der Verbindung des sauren Katalysators, bezogen auf die Ausgangsverbindung (X), eingesetzt. In einer Ausführungsform von Schritt 5) werden 2 bis 3 Äquivalente der Verbindung des sauren Katalysators, bezogen auf die Ausgangsverbindung (X), eingesetzt. In einer Ausführungsform von Schritt 5) werden 2 bis 5 Äquivalente Triethylorthoessigester, bezogen auf die Ausgangsverbindung (X), eingesetzt. In einer Ausführungsform von Schritt 5) werden 2 bis 4 Äquivalente Triethylorthoessigester, bezogen auf die Ausgangsverbindung (X), eingesetzt. In einer Ausführungsform von Schritt 5) werden 2 bis 3 Äquivalente Triethylorthoessigester, bezogen auf die Ausgangsverbindung (X), eingesetzt.

In einer Ausführungsform von Schritt 5) wird ein Lösungsmittel eingesetzt. In einer Ausführungsform von Schritt 5) wird ein Lösungsmittel ausgewählt aus Dimethylacetamid, NMP (N-Methyl-2-pyrrolidon), DMF (Dimethylformamid) und Mischungen davon eingesetzt. In einer Ausführungsform von Schritt 5) wird ein Lösungsmittel ausgewählt aus Dimethylacetamid, NMP (N-Methyl-2-pyrrolidon) und Mischungen davon eingesetzt. In einer Ausführungsform von Schritt 5) wird als Lösungsmittel Dimethylacetamid eingesetzt. In einer Ausführungsform von Schritt 5) wird als Lösungsmittel NMP (N-Methyl-2-pyrrolidon) eingesetzt.

In einer Ausführungsform von Schritt 5) wird der Schritt bei einer Temperatur von 80 bis 120 °C durchgeführt. In einer Ausführungsform von Schritt 5) wird der Schritt bei einer Temperatur von 100 bis 120 °C durchgeführt. In einer Ausführungsform von Schritt 5) wird der Schritt bei einer Temperatur von 90 bis 110 °C durchgeführt. In einer Ausführungsformvon Schritt 5) wird der Schritt bei einer Temperatur von 95 bis 105 °C durchgeführt. In einer Ausführungsform von Schritt 5) wird der Schritt bei einer Temperatur von 100 °C durchgeführt.

In einer Ausführungsform von Schritt 5) beträgt die Reaktionszeit zwischen 1 bis 4 Stunden. In einer Ausführungsform von Schritt 5) beträgt die Reaktionszeit zwischen 1 bis 3 Stunden. In einer Ausführungsform von Schritt 5) beträgt die Reaktionszeit zwischen 1,5 bis 2,5 Stunden. In einer Ausführungsform von Schritt 5) beträgt die Reaktionszeit 2 Stunden.

In einer Ausführungsform von Schritt 5) werden 2,5 bis 5 Äquivalente Triethylorthoessigester in Dimethylacetamid bei 100 bis 120°C für 1,5 bis 3 Stunden eingesetzt. In einer Ausführungsform von Schritt 5) werden 2,5 bis 5 Äquivalente Triethylorthoessigester in NMP (N-Methyl-2-pyrrolidon) bei 100 bis 120°C für 1,5 bis 3 Stunden eingesetzt.

In einer Ausführungsform umfasst das Verfahren zur Herstellung von Finerenone (II) die Schritte 1), 2), 3), 4), 5) und 6)

### Schritt 6) Verseifen der Verbindung der Formel (XI)

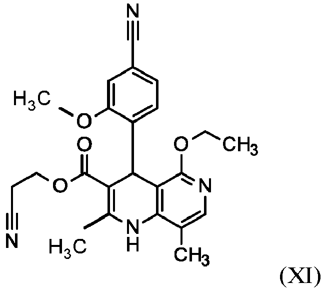

zu der Verbindung der Formel (XII)

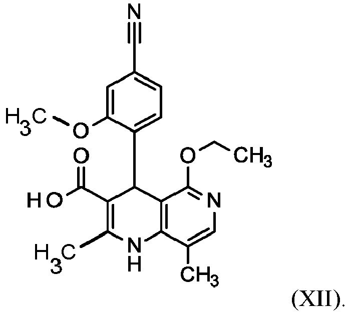

In einer Ausführungsform von Schritt 6) wird ein Lösungsmittel eingesetzt. In einer Ausführungsform von Schritt 6) wird ein Lösungsmittel ausgewählt aus Wasser, Tetrahydrofuran (THF), 2-Methyl-Tetrahydrofuran, Dioxan, 1,2-Dimethoxyethan und Mischungen davon eingesetzt. In einer Ausführungsform von Schritt 6) wird ein Lösungsmittel ausgewählt aus Wasser, Tetrahydrofuran (THF) und Mischungen davon eingesetzt. In einer Ausführungsform von Schritt 6) wird ein Lösungsmittelgemisch aus Wasser und Tetrahydrofuran (THF) eingesetzt.

In einer Ausführungsform von Schritt 6) ist das Lösungsmittel ein Lösungsmittelgemisch umfassend Wasser und einem Lösungsmittel, wobei das Volumenverhältnis von Lösungsmittel zu Wasser ausgewählt ist aus 0,2 bis 3 : 1 bis 2. In einer Ausführungsform von Schritt 6) ist das Lösungsmittel ein Lösungsmittelgemisch bestehend aus Wasser und einem Lösungsmittel, wobei das Volumenverhältnis von Lösungsmittel zu Wasser ausgewählt ist aus 0,4 bis 1,5 : 1.

In einer Ausführungsform von Schritt 6) ist das Lösungsmittel ein Lösungsmittelgemisch bestehend aus Wasser und THF, wobei das Volumenverhältnis von THF zu Wasser ausgewählt ist aus 0,4 bis 1,5 : 1. In einer Ausführungsform von Schritt 6) ist das Lösungsmittel ein Lösungsmittelgemisch bestehend aus Wasser und THF, wobei das Volumenverhältnis von THF zu Wasser ausgewählt ist aus 0,8 bis 1,5 : 1. In einer Ausführungsform von Schritt 6) ist das Lösungsmittel ein Lösungsmittelgemisch bestehend aus Wasser und THF, wobei das Volumenverhältnis von THF zu Wasser ausgewählt ist aus 1:1.

In einer Ausführungsform von Schritt 6) wird das Lösungsmittel in einer 6 bis 15-fachen Menge eingesetzt, wobei die Menge des Lösungsmittels die Volumenmenge in Mililiter (mL), bezogen auf 1 Gramm (g) eingesetztes Ausgangsmaterial (XI), ist. In einer Ausführungsform von Schritt 6) wird das Lösungsmittel in einer 7 bis 10-fachen Menge eingesetzt, wobei die Menge des Lösungsmittels die Volumenmenge in Mililiter (mL), bezogen auf 1 Gramm (g) eingesetztes Ausgangsmaterial (XI), ist. In einer Ausführungsform von Schritt 6) wird das Lösungsmittel in einer 8 bis 10-fachen Menge eingesetzt, wobei die Menge des Lösungsmittels die Volumenmenge in Mililiter (mL), bezogen auf 1 Gramm (g) eingesetztes Ausgangsmaterial (XI), ist. In einer Ausführungsform von Schritt 6) wird das Lösungsmittel in einer 7 bis 10-fachen Menge eingesetzt, wobei die Menge des Lösungsmittels die Volumenmenge in Mililiter (mL), bezogen auf 1 Gramm (g) eingesetztes Ausgangsmaterial (XI), ist und wobei das Lösungsmittel ausgewählt ist Wasser, THF und Mischungen davon. In einer Ausführungsform von Schritt 6) wird das Lösungsmittel in einer 8 bis 10-fachen Menge eingesetzt, wobei die Menge des Lösungsmittels die Volumenmenge in Mililiter (mL), bezogen auf 1 Gramm (g) eingesetztes Ausgangsmaterial (XI), ist und wobei das Lösungsmittel ausgewählt ist Wasser, THF und Mischungen davon.

In einer Ausführungsform von Schritt 6) erfolgt der Schritt in einem THF/Wasser Gemisch in einem Verhältnis von 0,6-2 :1 in einer 7 bis 10-fachen Menge. In einer Ausführungsform von Schritt 6) erfolgt der Schritt in einem THF/Wasser Gemisch in einem Verhältnis von 0,6-2:1 in einer 7 bis 10-fachen Menge unter Zusatz von einer Base. In einer Ausführungsform von Schritt 6) erfolgt der Schritt in einem THF/Wasser Gemisch in einem Verhältnis von 0,6-2 :1 in einer 7 bis 10-fachen Menge unter Zusatz von Natronlauge.

In einer Ausführungsform umfasst das Verfahren zur Herstellung von Finerenone (II) die Schritte 1), 2), 3), 4), 5), 6) und 7)

### Schritt 7) Umsetzen der Verbindung der Formel (XII)

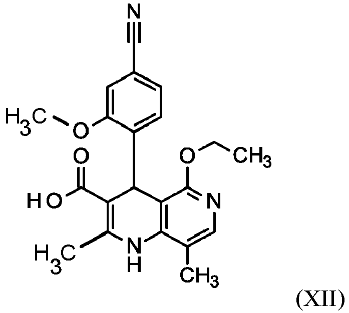

in THF mit Carbodiimidazol und 4-(Dimethylamino)-pyridin

In einer Ausführungsform von Schritt 7) wird ein Lösungsmittel ausgewählt aus THF, 2-Methyl-THF, Dioxan, 1,2-Dimethoxyethan und Mischungen davon eingesetzt. In einer Ausführungsform von Schritt 7) wird als Lösungsmittel THF eingesetzt. In einer Ausführungsform von Schritt 7) wird 4-(Dimethylamino)-pyridin in katalytischen Mengen eingesetzt. In einer Ausführungsform von Schritt 7) erfolgt dieser Schritt in einer Eintopfreaktion.

In einer Ausführungsform umfasst das Verfahren zur Herstellung von Finerenone (II) die Schritte 1), 2), 3), 4), 5), 6), 7) und 8)

### Schritt 8) Erhitzen des in Schritt 7) erhaltenen Produkts.

In einer Ausführungsform von Schritt 8) wird der Schritt unter Zugabe von Hexamethyldisilazan durchgeführt. In einer Ausführungsform von Schritt 8) wird der Schritt unter Zugabe von 3,0 bis 4,5 Äquivalenten Hexamethyldisilazan, bezogen auf das Ausgangsmaterial (XII), durchgeführt. In einer Ausführungsform von Schritt 8) wird der Schritt unter Zugabe von 3,2 bis 4 Äquivalenten Hexamethyldisilazan, bezogen auf das Ausgangsmaterial (XII), durchgeführt. In einer Ausführungsform von Schritt 8) wird für 16 bis 24 Stunden erhitzt. In einer Ausführungsform von Schritt 8) wird unter Zugabe von 3,0 bis 4,5 Äquivalenten Hexamethyldisilazan, bezogen auf das Ausgangsmaterial (XII), für 16 bis 24 Stunden erhitzt. In einer Ausführungsform von Schritt 8) wird der Schritt unter Zugabe von 3,2 bis 4 Äquivalenten Hexamethyldisilazan, bezogen auf das Ausgangsmaterial (XII), für 16 bis 24 Stunden erhitzt.

In einer Ausführungsform umfasst das Verfahren zur Herstellung von Finerenone (II) die Schritte 1), 2), 3), 4), 5), 6), 7), 8) und 9)

### Schritt 9) Hydrolyse des in Schritt 8) erhaltenen Produkts zu der Verbindung der Formel (XIII)

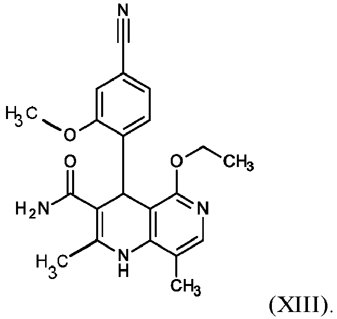

In einer Ausführungsform von Schritt 9) wird ein Lösungsmittel eingesetzt. In einer Ausführungsform von Schritt 8) ist das Lösungsmittel ausgewählt aus Wasser, THF, 2-Methyl-THF, Dioxan, 1,2-Dimethoxyethan und Mischungen davon.

In einer Ausführungsform von Schritt 9) ist das Lösungsmittel ein Lösungsmittelgemisch bestehend aus Wasser und THF. In einer Ausführungsform von Schritt 9) ist das Lösungsmittel ein Lösungsmittelgemisch bestehend aus Wasser und THF, wobei das THF in einer 3 bis 7-fachen Menge und Wasser in einer 0,2 bis 1-fachen Menge, eingesetzt wird, wobei die Menge des jeweiligen Lösungsmittels die Volumenmenge in Mililiter (mL), bezogen auf 1 Gramm (g) eingesetztes Ausgangsmaterial ist. In einer Ausführungsform von Schritt 9) ist das Lösungsmittel ein Lösungsmittelgemisch bestehend aus Wasser und THF, wobei das THF in einer 4 bis 6-fachen Menge und Wasser in einer 0,2 bis 0,8-fachen Menge, eingesetzt wird, wobei die Menge des jeweiligen Lösungsmittels die Volumenmenge in Mililiter (mL), bezogen auf 1 Gramm (g) eingesetztes Ausgangsmaterial ist. In einer Ausführungsform von Schritt 9) ist das Lösungsmittel ein Lösungsmittelgemisch bestehend aus Wasser und THF, wobei das THF in einer 4,5 bis 5,5-fachen Menge und Wasser in einer 0,3 bis 0,5-fachen Menge, eingesetzt wird, wobei die Menge des jeweiligen Lösungsmittels die Volumenmenge in Mililiter (mL), bezogen auf 1 Gramm (g) eingesetztes Ausgangsmaterial ist.

In einer Ausführungsform umfasst das Verfahren zur Herstellung von Finerenone (II)
- die Schritte 1), 2), und
- einen oder mehrere der Schritte ausgewählt aus Schritt 3), Schritt 4), Schritt 5), Schritt 6), Schritt 7), Schritt 8) und Schritt 9).

In einer Ausführungsform umfasst das Verfahren zur Herstellung von Finerenone (II) die Schritte 1), 2), und mindestens Schritt 4). In einer Ausführungsform umfasst das Verfahren zur Herstellung von Finerenone (II) die Schritte 1), 2), und mindestens Schritt 5). In einer Ausführungsform umfasst das Verfahren zur Herstellung von Finerenone (II) die Schritte 1), 2), und mindestens Schritt 6). In einer Ausführungsform umfasst das Verfahren zur Herstellung von Finerenone (II) die Schritte 1), 2), und mindestens Schritt 7). In einer Ausführungsform umfasst das Verfahren zur Herstellung von Finerenone (II) die Schritte 1), 2), und mindestens Schritt 8). In einer Ausführungsform umfasst das Verfahren zur Herstellung von Finerenone (II) die Schritte 1), 2), und mindestens Schritt 9).

Beispiele der Schritte 3) bis 9) sind in EP3174875 B1 beschrieben. Die Offenbarung der EP3174875B1 wird in die vorliegende Offenbarung inkludiert.

Ein anderer Aspekt der Erfindung ist die Verwendung des erfindungsgemäßen Verfahrens zur Herstellung von 4-Formyl-3-Methoxybenzonitril der Formel (I) 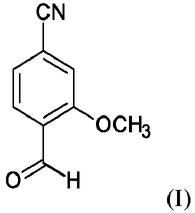 zur Herstellung von Finerenone nach Formel (II)

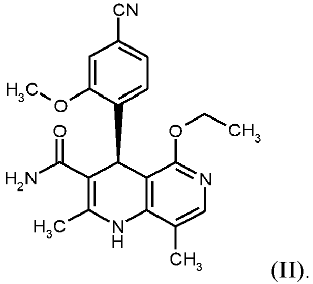

Ein anderer Aspekt der Erfindung ist die Verwendung von 4-Methyl-3-Methoxybenzonitril der Formel (III) 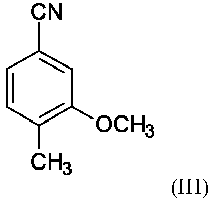 zur Herstellung von 4-Formyl-3-Methoxybenzonitril der Formel (I)

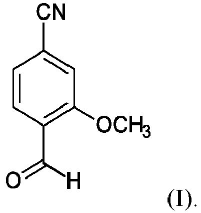

Ein anderer Aspekt der Erfindung ist die Verwendung von 3-Methoxy-4-(dibromomethyl)benzonitril der Formel (IV) 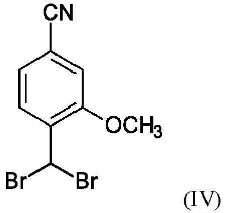 zur Herstellung von 4-Formyl-3-Methoxybenzonitril der Formel (I)

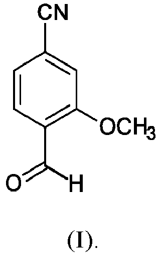

Ein anderer Aspekt der Erfindung ist die Verwendung von 4-Methyl-3-Methoxybenzonitril der Formel (III) 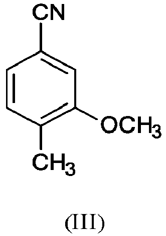 zur Herstellung von Finerenone nach Formel (II)

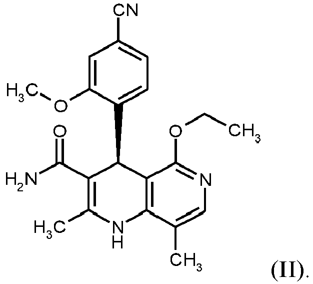

Ein anderer Aspekt der Erfindung ist die Verwendung von 3-Methoxy-4-(dibromomethyl)benzonitril der Formel (IV) 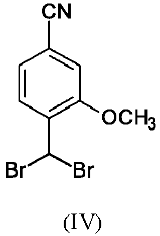 zur Herstellung von Finerenone nach Formel (II)

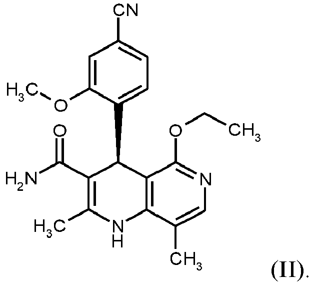

Ein weiterer Aspekt ist die Verwendung der Verbindung nach Formel (V), (Va) oder (Vb) 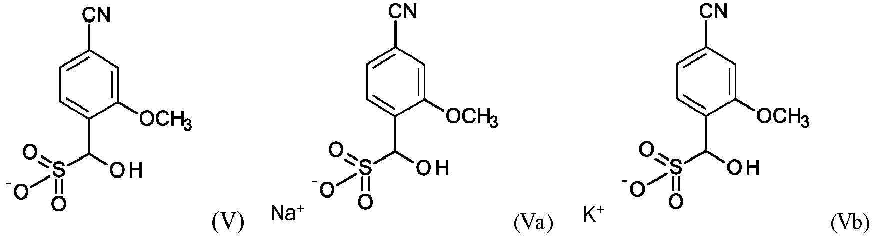 zur Herstellung von 4-Formyl-3-Methoxybenzonitril der Formel (I) oder Finerenone nach Formel (II)

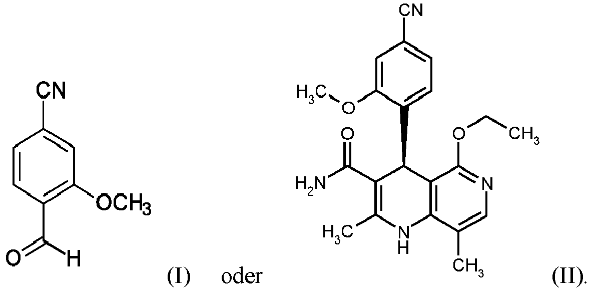

Ein weiterer Aspekt ist das Addukt nach Formel (V)

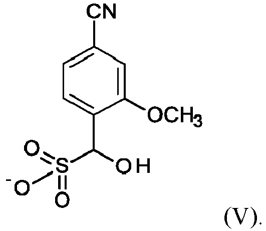

Ein weiterer Aspekt ist das Addukt nach Formel (V) 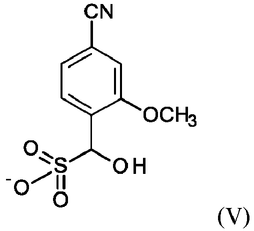 erhältlich durch Schritt (i) des Aufreinigungsschritts.

Im Folgenden sind weitere Ausführungsformen des erfindungsgemäßen Verfahrens wiedergegeben:
In einer Ausführungsform ist das Bromierungsreagenz in Schritt 1) ausgewählt ist aus N-Bromsuccinimid, 1,3-Dibrom-5,5-hydantoin, Brom und Mischungen davon. In einer Ausführungsform wird in Schritt 1) ein Lösungsmittel eingesetzt. In einer Ausführungsform wird in Schritt 1) ein Lösungsmittel ausgewählt aus Chloroform, Chlorbenzol, Dichlorbenzol und Mischungen davon eingesetzt. In einer Ausführungsform wird in Schritt 1) die Bromierung bei Temperaturen von 50 bis 120°C durchgeführt. In einer Ausführungsform wird in Schritt 1) ein Radikalstarter eingesetzt. In einer Ausführungsform wird in Schritt 1) ein Radikalstarter ausgewählt aus Dibenzoylperoxid und Azobisisobutyronitril eingesetzt. In einer Ausführungsform wird in Schritt 2) eine wässrige Hydrolyse durchgeführt. In einer Ausführungsform wird in Schritt 2) ein ist Lösungsmittel ausgewählt aus Wasser, Dimethylformamid, Dimethylacetamid, N-Methylpyrolidon (NMP), Tetramethylharnstoff, Chlorbenzol, Dichlorbenzol und Mischungen davon, eingesetzt. In einer Ausführungsform wird in Schritt 2) ein saurer Zusatz beigefügt. In einer Ausführungsform wird dem Reaktionsgemisch in Schritt 2) ein saurer Zusatz ausgewählt aus Salzsäure, Bromwasserstoffsäure, Schwefelsäure, Phosphorsäure, Ameisensäure, Essigsäure und Mischung davon beigefügt. In einer Ausführungsform wird in Schritt 2) dem Reaktionsgemisch ein basischer Zusatz beigefügt. In einer Ausführungsform wird in Schritt 2) dem Reaktionsgemisch in Schritt 2) ein basischer Zusatz ausgewählt aus Natriumhydroxid, Kaliumhydroxid, Calciumhydroxid, Natriumcarbonat, Natriumhydrogencarbonat, Kaliumcarbonat, Kaliumhydrogencarbonat, Natriumphosphat, Kaliumphosphat, Pyridin, Triethylamin, Ammoniak und Mischung davon beigefügt. In einer Ausführungsform wird in Schritt 2) die Hydrolyse bei Temperaturen von 80 bis 100°C erfolgt. In einer Ausführungsform wird in Schritt 2) beträgt die Reaktionszeit in Schritt 2) 2 bis 6 Stunden. In einer Ausführungsform wird nach Schritt 2) ferner einer oder mehrerer der folgenden Schritte ausgewählt aus Extraktionsschritt, Aufreinigungsschritt und Kristallisationsschritt durchgeführt.

In einer Ausführungsform wird nach Schritt 2) ferner eine intermediäre Aufreinigung von 4-Formyl-3-Methoxybenzonitril der Formel (I) umfassend die Schritte

### (iii) Umsetzung von 4-Formyl-3-Methoxybenzonitril der Formel (I) mit einer Bisulfitverbindung, wobei das Addukt nach Formel (V)

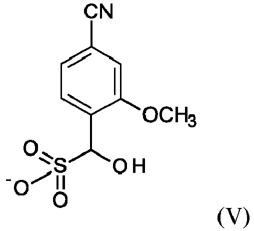

erhalten wird,

### (iv) basische Spaltung des Addukts nach Formel (V), wobei -Formyl-3-Methoxybenzonitrile der Formel (I)

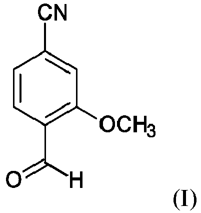

erhalten wird.

Eine Ausführungsform betrifft ein Verfahren zur Herstellung von Finerenone nach Formel (II) 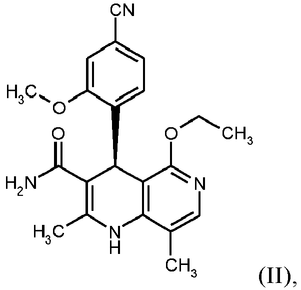 umfassend das Verfahren zur Herstellung von 4-Formyl-3-Methoxybenzonitril der Formel (I) 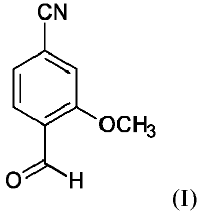 nach einer der Ausführungsformen wie weiter oben beschrieben.

In einer Ausführungsform kann eine oder mehrere der vorher ausgeführten Ausführungsformen zur Herstellung von 4-Formyl-3-Methoxybenzonitril der Formel (I) 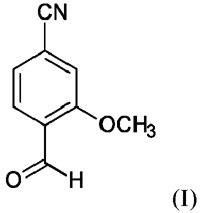 zur Herstellung von Finerenone nach Formel (II) 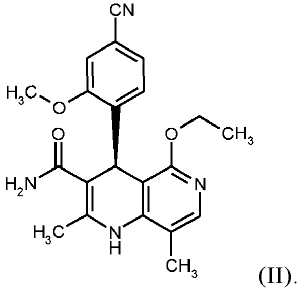 verwendet werden.

In einer Ausführungsform kann eine oder mehrere der vorher ausgeführten Ausführungsformen zur Herstellung von 4-Formyl-3-Methoxybenzonitril der Formel (I) oder von Finerenone nach Formel (II) 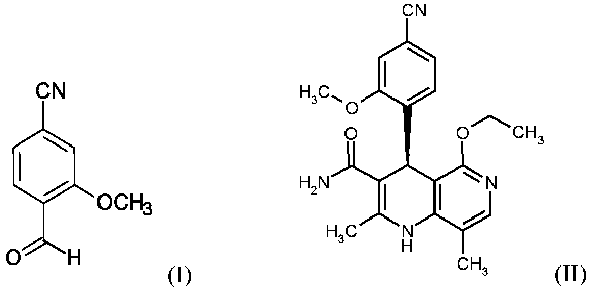 verwendet werden.

Die Erfindung betrifft eine Verbindung nach Formel (V), (Va) oder (Vb)

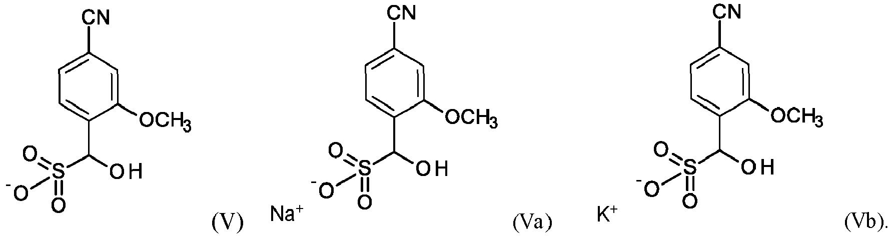

Die Erfindung betrifft die Verwendung der Verbindung nach Formel (V), (Va) oder (Vb) 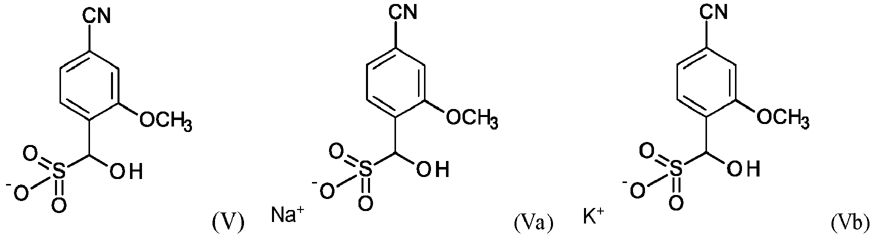 zur Herstellung von 4-Formyl-3-Methoxybenzonitril der Formel (I) oder Finerenone nach Formel (II)

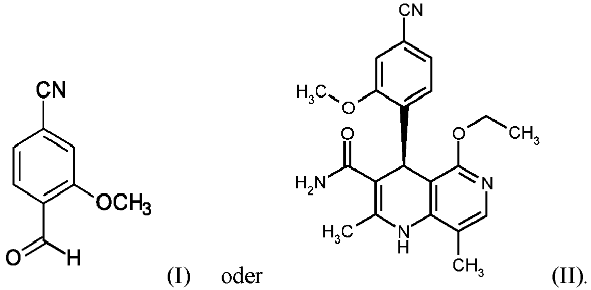

### Beispiele

**Abkürzungen**

| | |
|---|---|
| h | Stunden |
| g | Gramm |
| min | Minuten |
| °C | Grad Celsius |
| mL | Milliliter |
| mmol | Millimol |
| mol | Mol |
| aqu. | wä ssrig |
| d. Th. | der Theorie |

### Beispiel 1: 4-Formyl-3-methoxybenzonitril der Formel (I)

Eine Mischung aus 100,00 g (679,44 mmol) 4-Methyl-3-Methoxybenzonitril (III), 213,69 g (747,38 mmol) 1,3-Dibrom-5,5-hydantoin. und 5,58 g (33,97 mmol) Azobisisobutyronitril in 800 mL Chlorbenzol wurden 4 h lang bei 90°C erhitzt. Man kühlte auf 20°C ab, gab 200 mL Wasser zu und rührte 1 h bei 20°C. Anschließend wurde der ausgefallene Feststoff abfiltriert und die Phasen getrennt. Die Chlorbenzol -Phase wurde auf 400 mL Endvolumen abdestilliert. Man gab 400 mL Wasser und 34,25 g (407,66 mmol) Natriumhydrogencarbonat zu und rührte anschließend 4 h unter Rückfluss (intensives Rühren des Zwei-Phasen-Gemisches). Man kühlte die Lösung ab, die Chlorbenzol-Phase wurde abgetrennt, einmal mit 200 mL gesättigter aqu. Natriumcarbonat-Lösung gewaschen und anschließend weitgehend (bis zur Rührbarkeitsgrenze) eingeengt (unter Vakuum). Man gab 800 mL Heptan zu und kühlte auf 0°C ab und impfte mit 4-Formyl-3-Methoxybenzonitril (I)4-Formyl-3-Methoxybenzonitril (I)an, rührte 4 h bei 0°C nach. Man filtrierte die ausgefallenen Kristalle ab und trocknete anschließend bei 45°C unter Vakuum (20 mbar / Stickstoff Schleppgas).

Ausbeute: 82,67 g (75,5 % d. Th. über zwei Stufen), Reinheit (HPLC 100% Methode) > 99,3 %, MS (EIpos): m/z = 162 [M⁺H]⁺ , ¹H-NMR (300 MHz, DMSO-d6): δ =3.97 (s, 3H), 7.53 (d, 1H), 7.80 (s, 1H), 7.81 (d, 1H), 10.36(s, 1H).

### Beispiel 2: 4-Formyl-3-methoxybenzonitril der Formel (I)

Eine Mischung aus 100,00 g (679,44 mmol) 4-Methyl-3-Methoxybenzonitril 4-Methyl-3-Methoxybenzonitril (III), 266,05 g (1495,00 mmol) N-Bromsuccinimid und 8,22 g (33,97 mmol) Dibenzoylperoxid in 800 mL Chloroform wurden 4 h lang unter Rückfluss erhitzt. Man kühlte auf 20°C ab, gibt 200 mL Wasser zu und rührte 1 h bei 20°C. Anschließend wurde das ausgefallene Succinimid abfiltriert und die Phasen getrennt. Die Chloroform-Phase wurde auf 400 mL Endvolumen abdestilliert. Man gab 400 mL Wasser und rührte anschließend 4 h unter Rückfluss (intensives Rühren des Zwei-Phasen-Gemisches). Man kühlte die Lösung ab, trennte die wässrige Phase ab, stellte den pH-Wert durch Zugabe von 10%iger aqu. Natronlauge auf pH 7,0 und wusch anschließend die organische Phase einmal mit einer 5%igenNatriumsulfit-Lösung. Die Chloroform-Phase wurde abgetrennt und weitgehend (bis zur Rührbarkeitsgrenze) eingeengt (unter Vakuum). Man gab 400 mL Essigester zu und destillierte auf ca. 150 mL Volumen ab (unter Vakuum). Man kühlte auf -15°C ab und impfte die übersättigte Lösung mit 0,5 g 4-Formyl-3-Methoxybenzonitril (I) 4-Formyl-3-Methoxybenzonitril (I) an, rührte 4 h bei -15°C. Man filtrierte die ausgefallenen Kristalle ab und trocknete anschließend bei 45°C unter Vakuum (20 mbar / Stickstoff Schleppgas).

### Ausbeute: 81,80 g (74,7 % d. Th. über zwei Stufen), Reinheit (HPLC 100% Methode) > 99,2%

### Beispiel 3: 4-Formyl-3-methoxybenzonitril der Formel (I)

Eine Mischung aus 100,00 g (679,44 mmol) 4-Methyl-3-Methoxybenzonitril 4-Methyl-3-Methoxybenzonitril(III), 8,22 g (33,97 mmol) Dibenzoylperoxid und 213,69 g (747,38 mmol) 1,3-Dibrom-5,5-hydantoin in 1000 mL Chlorbenzol wurden 4 h lang bei 90°C erhitzt. Man kühlte auf 20°C ab, gab 200 mL einer 10%igen wässrigen Natriumsulfit-Lösung zu und rührte 1 h bei 20°C. Anschließend wurde der ausgefallene Feststoff abfiltriert und die Phasen getrennt. Die Chlorbenzol-Phase wurde bis auf Rührbarkeitsgrenze abdestilliert. Man gab 500 mL Dimethylformamid zu und destillierte auf ca. 250 mL Endvolumen im Vakuum das Lösungsmittel ab. Man gab 250 mL Wasser zu und rührte anschließend 1 h bei 90°C. Man kühlte die Lösung auf 20°C ab, gab 500 mL Methylisobutylketon (MIBK) zu und rührte 15 min bei 20°C. Die organische Phase wurde abgetrennt und mit 500 mL einer gesättigten Natriumhydrogencarbonat-Lösung gewaschen. Anschließend wurde mit 250 mL Wasser gewaschen. Die organische Phase wurde bis zur Rührbarkeitsgrenze im Vakuum eingeengt und 600 mL Heptan zugegeben. Man kühlte auf 0°C ab und impfte mit 0,5 g 4-Formyl-3-Methoxybenzonitril (I) an, und rührte 4 h bei 0°C weiter. Man filtrierte die ausgefallenen Kristalle ab und trocknete anschließend bei 45°C unter Vakuum (20 mbar / Stickstoff Schleppgas).

### Ausbeute: 83,33 g (76,1 % d. Th. über zwei Stufen), Reinheit (HPLC 100% Methode) > 99,1%

### Beispiel 4: 4-Formyl-3-methoxybenzonitril der Formel (I)

Eine Mischung aus 100,00 g (679,44 mmol) 4-Methyl-3-Methoxybenzonitril (III), 266,05 g (1495,00 mmol) N-Bromsuccinimid und 8,22 g (33,97 mmol) Dibenzoylperoxid in 800 mL Chloroform wurden 4 h lang unter Rückfluss erhitzt. Man kühlte auf 20°C ab, gibt 200 mL Wasser zu und rührte 1 h bei 20°C. Anschließend wurde das ausgefallene Succinimid abfiltriert und die Phasen getrennt. Die ChloroformPhase wurde auf 400 mL Endvolumen abdestilliert. Man gab 400 mL Wasser zu und rührte anschließend 4 h unter Rückfluss (intensives Rühren des Zwei-Phasen-Gemisches). Man kühlte die Lösung ab, trennte die wässrige Phase ab, stellte den pH-Wert durch Zugabe von 10%iger aqu. Natronlauge auf pH 7,0 und wusch anschließend die organische Phase einmal mit einer 5%igen Natriumsulfit-Lösung. Die Chloroform-Phase wurde abgetrennt und weitgehend (bis zur Rührbarkeitsgrenze) eingeengt (unter Vakuum). Man gibt 400 mL Essigester zu und destillierte auf ca. 100 mL Volumen ab (unter Vakuum). Man gab 600 mL Heptan zu, destillierte anschließend 100 mL Lösungsmittel unter Vakuum ab, impfte mit 0,5 g 4-Formyl-3-Methoxybenzonitril (I) an, rührte 4 h bei 0°C. Man filtrierte die ausgefallenen Kristalle ab und trocknete anschließend bei 45°C unter Vakuum (20 mbar / Stickstoff Schleppgas).

### Ausbeute: 80,92 g (73,9 % d. Th. über zwei Stufen), Reinheit (HPLC 100% Methode) > 99,3 %

### Beispiel 5: 4-Formyl-3-methoxybenzonitril der Formel (I)

Eine Mischung aus 100,00 g (679,44 mmol) 4-Methyl-3-Methoxybenzonitril (III), 213,69 g (747,38 mmol) 1,3-Dibrom-5,5-hydantoin. und 5,58 g (33,97 mmol) Azobisisobutyronitril in 800 mL Chlorbenzol wurden 4 h lang bei 90°C erhitzt. Man kühlte auf 20°C ab, gab 200 mL Wasser zu und rührte 1 h bei 20°C. Anschließend wurde der ausgefallene Feststoff abfiltriert und die Phasen getrennt. Die Chlorbenzol -Phase wurde auf 400 mL Endvolumen abdestilliert. Man gab 400 mL Wasser zu und rührte anschließend 4 h unter Rückfluss (intensives Rühren des Zwei-Phasen-Gemisches). Man kühlte die Lösung ab, die Chlorbenzol-Phase wurde abgetrennt, anschließend wurde mit 200 mL gesättigter aqu. Natriumcarbonat-Lösung gewaschen und weitgehend (bis zur Rührbarkeitsgrenze) eingeengt (unter Vakuum). Man gab 400 mL Essigester und 800 mL Heptan zu und destillierte 500 mL Lösungsmittel im Vakuum ab, kühlte auf 0°C ab und impfte mit 0,5 g 4-Formyl-3-Methoxybenzonitril (I) an, rührte 4 h bei 0°C. Man filtrierte die ausgefallenen Kristalle ab und trocknete anschließend bei 45°C unter Vakuum (20 mbar / Stickstoff Schleppgas).

### Ausbeute: 82,02 g (74,9 % d. Th. über zwei Stufen), Reinheit (HPLC 100% Methode) > 99,2 %

### Beispiel 6: 4-Formyl-3-methoxybenzonitril der Formel (I)

Eine Mischung aus 100,00 g (679,44 mmol) 4-Methyl-3-Methoxybenzonitril (III), 213,69 g (747,38 mmol) 1,3-Dibrom-5,5-hydantoin. und 5,58 g (33,97 mmol) Azobisisobutyronitril in 800 mL Chlorbenzol wurden 4 h lang bei 90°C erhitzt. Man kühlte auf 20°C ab, gab 200 mL Wasser zu und rührte 1 h bei 20°C. Anschließend wurde der ausgefallene Feststoff abfiltriert und die Phasen getrennt. Die Chlorbenzol -Phase wird auf 400 mL Endvolumen abdestilliert. Man gab 400 mL Wasser und 20 mL konz. Salzsäure zu rührte anschließend 4 h unter Rückfluss (intensives Rühren des Zwei-Phasen-Gemisches). Man kühlte die Lösung ab, trennte die organische Phase ab und wusch diese mit 300 mL einer wässrigen 5%igen aqu. Natriumcarbonat-Lösung. Die Chlorbenzol-Phase wurde abgetrennt und weitgehend (bis zur Rührbarkeitsgrenze) eingeengt (unter Vakuum). Man gab 800 mL Heptan zu und kühlte auf 0°C ab und impfte mit 0,5 g 4-Formyl-3-Methoxybenzonitril(1) an, rührte 4h bei 0°C. Man filtrierte die ausgefallenen Kristalle ab und trocknete anschließend bei 45°C unter Vakuum (20 mbar/ Stickstoff Schleppgas).

### Ausbeute: 82,56 g (75,4 % d. Th. über zwei Stufen), Reinheit (HPLC 100% Methode) > 99,2 %

### Beispiel 7: 4-Formyl-3-methoxybenzonitril der Formel (I)

Eine Mischung aus 100,00 g (679,44 mmol) 4-Methyl-3-Methoxybenzonitril (III), 266,05 g (1495,00 mmol) N-Bromsuccinimid und 8,22 g (33,97 mmol) Dibenzoylperoxid in 800 mL Chloroform wurden 4 h lang unter Rückfluss erhitzt. Man kühlte auf 20°C ab, gibt 200 mL Wasser zu und rührte 1 h bei 20°C. Anschließend wurde das ausgefallene Succinimid abfiltriert und die Phasen getrennt. Die ChloroformPhase wurde auf 400 mL Endvolumen abdestilliert. Man gab 400 mL Wasser zu und rührte anschließend 4 h unter Rückfluss (intensives Rühren des Zwei-Phasen-Gemisches). Man kühlte die Lösung ab und trennte die organische Phase ab. Die Chloroform-Phase wurde weitgehend (bis zur Rührbarkeitsgrenze) eingeengt (unter Vakuum). Anschließend wurde ein Bisulfit-Addukt zur Aufreinigung des Aldehyds (I) wie folgt hergestellt: Man gab 83,25 g (800 mmol) Natriumhydrogensulfit in 400 mL Wasser. Unterstarkem Rühren wurde die Chloroformphase langsam zugetropft und destillierte dabei unter Vakuum Chloroform ab. Der ausgefallene Feststoff (Bisulfit-Addukt) wurde abfiltriert und mit zweimal mit je 300 mL Wasser gewaschen. Der Feststoff wurde in 400 mL Wasser suspendiert und mit 500 mL Essigester versetzt. Unter Rühren stellte man den pH-Wert durch Zugabe von 30% iger aqu. Natronlauge auf pH 9,5. Die Essigester-Phase wurde abgetrennt, einmal mit 100 mL 1 N Salzsäure gewaschen. Man gab 200 mL Essigester zu und destillierte auf ca. 150 mL Volumen ab (unter Vakuum). Man kühlte auf -15°C ab und impfte die übersättigte Lösung mit 0,5 g 4-Formyl-3-Methoxybenzonitril (I) an, rührte 4 h bei -15°C. Man filtrierte die ausgefallenen Kristalle ab und trocknete anschließend bei 45°C unter Vakuum (20 mbar/ Stickstoff Schleppgas).

### Ausbeute: 78,07 g (71,3 % d. Th. über zwei Stufen), Reinheit (HPLC 100% Methode) > 99,3 %

### Beispiel 8: Kristallisationsvorschrift zur Aufreinigung des Aldehyds der Formel (I)

85,00 g 4-Formyl-3-Methoxybenzonitril (I), roh (Reinheit laut HPLC ca. 97,5 %) wurden in 215 mL einer Mischung aus MIBK (Methyl-Isobutyl-Keton) und n-Heptan (1:2) durch Erhitzen auf Rückfluss (~ 95°C) gelöst. Es wurde für 1 h bei Reflux gerührt. Nach 1 h war das Material vollständig aufgelöst; Die Lösung wurde langsam auf Raumtemperatur (in ~ 4h bis 23 ° C) abgekühlt, bei 75 ° C startete Fällung des Produktes. Dann wurde die Suspension für 1 h bei Raumtemperatur (~ 23°C) gerührt und durch Filtration isoliert. Das Produkt wurde mit 40 mL einer Mischung aus MIBK/n-Heptan 1:2 gewaschen und anschließend unter Vakuum bei 45 °C (20 mbar) über Nacht getrocknet.

### Ausbeute: 73,6 g (86.6 % d. Th.), Reinheit (HPLC 100% Methode) > 99,7 %

### Beispiel 9: Aufreinigung 3-Methoxy-4-(dibromomethyl)benzonitril

Eine kleine Probe 3-Methoxy-4-(dibromomethyl)benzonitril (IV) wurde mittels Chromatographie an Kieselgel (Laufmittel: Heptan/Ethylacetat = 15:1) aufgereinigt und spektroskopisch analysiert:
1HNMR (300 MHz in DMSO) δ : 7.88 (d, J ) 8.0 Hz, 1H), 7.62 (d, J ) 1.4 Hz, 1H), 7.53 (dd, J ) 8.0 and 1.5 Hz, 1H), 7.35 (s, 1H), 3.97 (s, 3H)

## Patentansprüche

1. Verfahren zur Herstellung von 4-Formyl-3-Methoxybenzonitril der Formel (I), 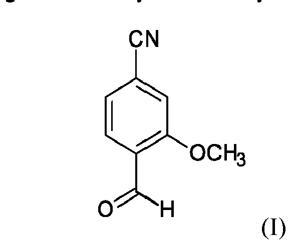 umfassend die Schritte
Schritt 1) Umsetzen von 4-Methyl-3-Methoxybenzonitril der Formel (III) 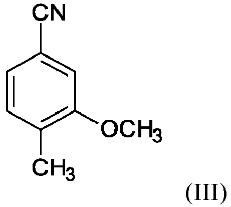 mit einem Bromierungsreagenz, wobei 3-Methoxy-4-(dibromomethyl)benzonitril der Formel (IV) 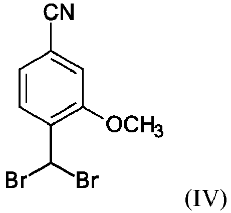 erhalten wird,
Schritt 2) Hydrolyse von 3-Methoxy-4-(dibromomethylbenzonitril) der Formel (IV) 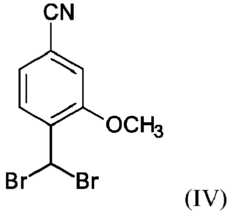 zu der Verbindung der Formel (I)

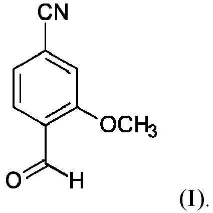

2. Verfahren nach Anspruch 1, wobei das Bromierungsreagenz in Schritt 1) ausgewählt ist aus N-Bromsuccinimid, 1,3-Dibrom-5,5-hydantoin, Brom und Mischungen davon.

3. Verfahren nach Anspruch 1 oder 2, wobei in Schritt 1) ein Lösungsmittel ausgewählt aus Chloroform, Chlorbenzol, Dichlorbenzol und Mischungen davon eingesetzt wird.

4. Verfahren nach einem der Ansprüche 1 bis 3, wobei die Bromierungin Schritt 1) bei Temperaturen von 50 bis 120°C durchgeführt wird.

5. Verfahren nach einem der Ansprüche 1 bis 4, wobei in Schritt 1) ein Radikalstarter ausgewählt aus Dibenzoylperoxid und Azobisisobutyronitril eingesetzt wird.

6. Verfahren nach einem der Ansprüche 1 bis 5, wobei in Schritt 2) eine wässrige Hydrolyse durchgeführt wird.

7. Verfahren nach einem der Ansprüche 1 bis 6, wobei in Schritt 2) ein ist Lösungsmittel ausgewählt aus Wasser, Dimethylformamid, Dimethylacetamid, N-Methylpyrolidon (NMP), Tetramethylharnstoff, Chlorbenzol, Dichlorbenzol und Mischungen davon, eingesetzt wird.

8. Verfahren nach einem der Ansprüche 1 bis 7, wobei dem Reaktionsgemisch in Schritt 2) ein saurer Zusatz ausgewählt aus Salzsäure, Bromwasserstoffsäure, Schwefelsäure, Phosphorsäure, Ameisensäure, Essigsäure und Mischung davon beigefügt wird.

9. Verfahren nach einem der Ansprüche 1 bis 8, wobei dem Reaktionsgemisch in Schritt 2) ein basischer Zusatz ausgewählt aus Natriumhydroxid, Kaliumhydroxid, Calciumhydroxid, Natriumcarbonat, Natriumhydrogencarbonat, Kaliumcarbonat, Kaliumhydrogencarbonat, Natriumphosphat, Kaliumphosphat, Pyridin, Triethylamin, Ammoniak und Mischung davon beigefügt wird.

10. Verfahren nach einem der Ansprüche 1 bis 9, wobei die Hydrolyse in Schritt 2) bei Temperaturen von 80 bis 100°C erfolgt.

11. Verfahren nach einem der Ansprüche 1 bis 10, wobei die Reaktionszeit in Schritt 2) 2 bis 6 Stunden beträgt.

12. Verfahren nach einem der Ansprüche 1 bis 11, wobei das Verfahren nach Schritt 2) ferner eine intermediäre Aufreinigung von 4-Formyl-3-Methoxybenzonitril der Formel (I) umfassend die Schritte
(v) Umsetzung von 4-Formyl-3-Methoxybenzonitril der Formel (I) mit einer Bisulfitverbindung, wobei das Addukt nach Formel (V) 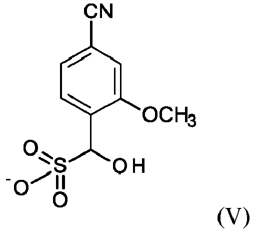 erhalten wird,
(vi) basische Spaltung des Addukts nach Formel (V), wobei -Formyl-3-Methoxybenzonitrile der Formel (I) 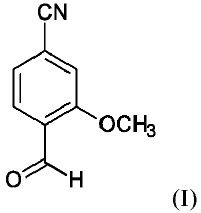 erhalten wird.

13. Verfahren zur Herstellung von Finerenone (II) nach Formel (II) 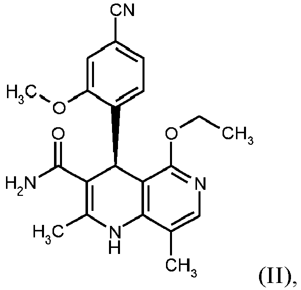 umfassend Schritt 1) und Schritt 2) zur Herstellung von 4-Formyl-3-Methoxybenzonitril der Formel (I) nach einem der Ansprüche 1 bis 12.

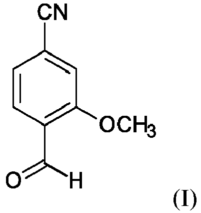

14. Verwendung des Verfahrens nach einem der Ansprüche 1 bis 12 zur Herstellung von 4-Formyl-3-Methoxybenzonitril der Formel (I) 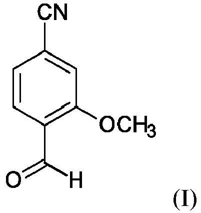 zur Herstellung von Finerenone nach Formel (II)

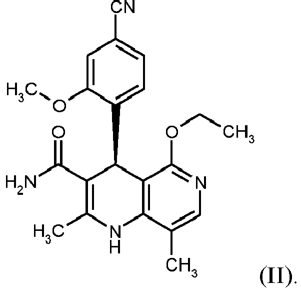

15. Verwendung von 4-Methyl-3-Methoxybenzonitril der Formel (III) oder 3-Methoxy-4-(dibromomethyl)benzonitril der Formel (IV) 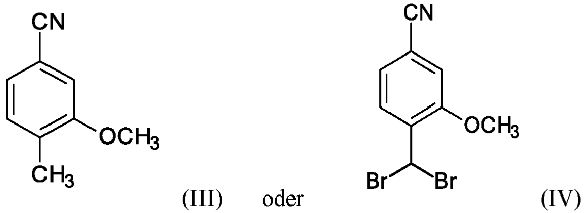 zur Herstellung von 4-Formyl-3-Methoxybenzonitril der Formel (I) oder Finerenone nach Formel (Π)
